# EUROPÄISCHE PATENTSCHRIFT

(11) **EP 1 112 362 B1**
(45) Veröffentlichungstag und Bekanntmachung des Hinweises auf die Patenterteilung: **08.02.2006**
(21) Anmeldenummer: 99947277.2
(22) Anmeldetag: 06.09.1999
(51) Int. Cl.: C12N 15/12, C07K 14/47, C12Q 1/68, C12N 15/11, A61K 31/70, A61K 48/00, C07K 16/18, G01N 33/50, A01K 67/027

(54) **GENVERÄNDERUNG IM GEN FÜR DIE G$g(b)3-UNTEREINHEIT DES HUMANEN G-PROTEINS**
GENE MODIFICATION IN THE GENE FOR THE DIE G$g(b)3-SUB-UNIT OF HUMAN G-PROTEIN
MODIFICATION GENIQUE DANS LE GENE DE LA SOUS-UNITE G$g(b)3 DE LA PROTEINE G HUMAINE

(30) Priorität: 10.09.1998 DE 19841299; 05.02.1999 DE 19904825; 18.03.1999 DE 19912049; 29.03.1999 DE 19914229; 30.04.1999 DE 19919989; 21.05.1999 DE 19923539
(43) Veröffentlichungstag der Anmeldung: 04.07.2001
(73) Patentinhaber: Siffert, Winfried, 45147 Essen (DE)
(72) Erfinder: Siffert, Winfried, Prof. Dr., 45147 Essen (DE)
(74) Vertreter: Cohausz & Florack
(86) Internationale Anmeldenummer: PCT/EP1999/006534
(87) Internationale Veröffentlichungsnummer: WO 2000/015785

(56) Entgegenhaltungen:
- WO-A-97/43442
- WO-A-98/11212
- US-A- 5 766 851
- ZHANG Y. ET AL.: "UKPDS 19: Heterogeneity in NIDDM: Separate contributions of IRS-1 and beta-3-adrenergic-receptor mutations to insulin resistance and obesity respectively with no evidence for glycogen synthase gene mutations." DIABETOLOGIA, Bd. 39, Nr. 12, 1996, Seiten 1505-1511, XP000892144 ISSN: 0012-186X
- HOPES E. ET AL.: "Association of glutamine 27 polymorphism of beta2 adrenoceptor with reported childhood asthma: Population based study." BMJ, Bd. 316, Nr. 7132, 28. Februar 1998 (1998-02-28), Seite 664 XP002137177 ISSN: 0959-8138
- YOSHIMURA MICHIHIRO ET AL.: "A missense Glu298Asp variant in the endothelial nitric oxide synthase gene is associated with coronary spasm in the Japanese." HUMAN GENETICS, Bd. 103, Nr. 1, Juli 1998 (1998-07), Seiten 65-69, XP000905545 ISSN: 0340-6717
- MUMMIDI S. ET AL.: "Genealogy of the CCR5 locus and chemokine system gene variants associated with altered rates of HIV-1 disease progression" NATURE MEDICINE, Bd. 4, Nr. 7, Juli 1998 (1998-07), Seiten 786-793-793, XP002121126 in der Anmeldung erwähnt
- VAN RIJ R. P. ET AL.: "The role of a stromal cell-derived factor-1 chemokine gene variant in the clinical course of HIV-1 infection." AIDS, Bd. 12, Nr. 9, 18. Juni 1998 (1998-06-18), Seiten F85-F90, XP000906934 ISSN: 0269-9370
- NABER C. ET AL.: "Genetic variability of G-protein reactivity as risk-factor for coronary artery disease and myocardial infarction." JOURNAL OF MOLECULAR MEDICINE, Bd. 76, Nr. 6, Mai 1998 (1998-05), Seite B28 XP000905343 ISSN: 0946-2716
- SPIEGEL A. M.: "Inborn errors of signal transduction: Mutations in G proteins and G protein-coupled receptors as a cause of disease." JOURNAL OF INHERITED METABOLIC DISEASE, Bd. 20, Nr. 2, 1997, Seiten 113-121, XP000905450 ISSN: 0141-8955
- SIFFERT W. ET AL.: "Association of a human G-protein beta3 subunit variant with hypertension." NATURE GENETICS, Bd. 18, Nr. 1, Januar 1998 (1998-01), Seiten 45-48, XP000892164 ISSN: 1061-4036

## Beschreibung

Die vorliegende Erfindung betrifft eine neue Nukleinsäuresequenz codierend für die Gβ3-Untereinheit des humanen G-Proteins, sowie die Verwendung von Gβ3-Untereinheiten der G-Proteine zur Ermittlung des Risikos, an einer Krankheit, die mit einer G-Protein-Fehlsteuerung assoziiert ist, zu erkranken.

Heterotrimere Guaninnukleotid-bindende Proteine (G-Proteine) haben eine herausragende Bedeutung bei der intrazellulären Signaltransduktion. Sie vermitteln die Weiterleitung extrazellulärer Signale nach Stimulation von Hormonrezeptoren und anderen Rezeptoren, welche nach Rezeptoraktivierung eine Konformationsänderung durchmachen. Dies führt zur Aktivierung von G-Proteinen, welche nachfolgend intrazelluläre Effektoren (z.B. Ionenkanäle, Enzyme) aktivieren oder hemmen können. G-Proteine steuern die intrazelluläre Signalverabeitung nach hormoneller Stimulation heptahelikaler Rezeptoren in der Zellmembran, aber auch nach Stimulation von Rezeptoren mit intrinsischer Tyrosinkinaseaktivität. Zu den regulierten Zellfunktionen gehören unter anderem Zellteilung und Zellwachstum, Kontraktion, Freisetzung von Zellinhaltsstoffen u.v.m..

Heterotrimere G-Proteine sind aus drei Untereinheiten, den α-, β- und γ-Untereinheiten, zusammengesetzt. Bislang wurden mehrere unterschiedliche α-Untereinheiten, 5 β-Untereinheiten und ca. 12 γ-Untereinheiten mittels biochemischer und molekularbiologischer Methoden nachgewiesen (Birnbaumer, L. and Birnbaumer, M., signaltransduction by G proteins: 1994 edition. J.Recept.Res. 15:213-252,1995; Offermans, S. and Schultz, G. Complex information processing by the transmembrane signaling system involving G proteins. Naunyn Schmiedebergs Arch.Pharmacol. 350:329-338,1994; Nürnberg, B., Gudermann, T. and Schultz, G. Receptors and G proteins as primary components of transmembrane signal transduction. Part 2. G proteins: structure and function. J.Mol.Med. 73:123-132,1995; Neer, E.J. Heterotrimeric G proteins: Organizers of Transmembrane Signals. Cell 80:249-257, 1995; Rens-Domiano, S. and Hamm, H.E. Structural and functional relationships of heterotrimeric G proteins. FASEB J. 9:1059-1066, 1995).

Die rezeptorvermittelte Aktivierung bestimmter α-Untereinheiten kann durch Vorbehandlung mit Pertussistoxin (PTX) gehemmt werden. Dazu gehören insbesondere die α-Isoformen α-i1, α-i2 und α-i3 sowie unterschiedliche αo-Untereinheiten. Solche G-Proteine werden auch als "PTXsensitive G-Proteine bezeichnet.

βγ-Untereinheiten erfüllen wesentliche Funktionen bei der G-Protein-Aktivierung sowie bei der Modulation intrazellulärer Reaktionen. Alle bisher bekannten G-Protein-β-Untereinheiten weisen auf der Ebene der Nukleodtidsequenz und auf der Ebene der Aminosäuresequenz hohe Homologien auf. Dabei werden diese Ähnlichkeiten nicht nur innerhalb der humanen β-Untereinheiten (Gβ1, Gβ2, Gβ3) gefunden, sondern auch im Vergleich zu β-Untereinheiten anderer Spezies, beispielsweise Fruchtfliege oder Hefe.

Kürzlich konnte im humanen GNB3-Gen, das für die Gβ3-Untereinheit kodiert, eine Basenveränderung in Exon 10 (C825T) beschrieben werden, die zum alternativen Spleißen von Exon 9 führt. Das alternative Spleißen wird durch eine kryptische Spleißstelle in Exon 9 begünstigt, wobei der entfernt liegende Basenaustausch C825T das Spleißen verstärkt. Das alternative Spleißprodukt (Gβ3s) weist einen Verlust von 123 bp (= 41 Aminosäuren) auf. Das GNB3-825T-Allel ist mit gesteigerter Aktivierbarkeit von G-Proteinen und der essentiellen Hypertonie assoziiert (Siffert, W., Rosskopf, D., Siffert, G., Busch, S., Moritz, A., Erbel, R., Sharma, A.M., Ritz, E., Wichmann, H.E., Jakobs, K.H., and Horsthemke, B. Association of a human G-protein beta3 subunit variant with hypertension. Nat.Genet. 18(1):45-48, 1998; Clapham, D.E. and Neer, E.J. G protein βγ subunits. Annu.Rev.Pharmacol.Toxicol. 37:167-203, 1997; Hamm, H.E. and Gilchrist, A. Heterotrimeric G proteins. Curr.Opin.Cell Biol. 8:189-196, 1996).

Dieses humane GNB3-Gen ist von Levine et al. (Levine,M.A., Smallwood,P.M., Moen,P.T. Jr., Helman,L.J. und Ahn,T.G. Molecular cloning of beta 3 subunit, a third form of the G protein beta-subunit polypeptide. Proc. Natl. Acad. Sci. U.S.A. 87 (6), 2329-2333 (1990)) beschrieben worden.

Gegenstand der Erfindung ist eine neue humane cDNA für die Gβ3-Untereinheit von humanem G-Protein.

Überraschenderweise hat es sich nämlich herausgestellt, daß es im humanen GNB3-Gen einen weiteren Polymorphismus gibt, der im folgenden "C1429T" genannt wird. Dieser Polymorphismus findet sich an Position 1429 der cDNA. Dies entspricht dem Exon 11 der prä-mRNA, jedoch außerhalb des offenen Leserahmens im 3' nichttranslatierten Bereich.

Dieser Polymorphismus steht in einem ausgeprägten Verteilungsungleichgewicht mit dem bekannten C825T-Polymorphismus- vgl. WO 97 43442 A, derart, daß nahezu alle GBN3-825C-Allele den Genotyp 1429C und nahezu alle GBN3-825T-Allele den Genotyp 1429T aufweisen. Somit eignet sich dieser Polymorphismus C1429T ebensogut wie der Polymorphismus C825T zum Nachweis der gesteigerten Aktivierbarkeit von G-Proteinen.

Der Polymorphismus C1429T ist bezogen auf die Sequenz der cDNA, wie sie von Levine und Mitarbeitern beschrieben wurde. Hierbei kommt es an Position 1429 der cDNA zu einem Austausch des C durch ein T:

1381 ggcctgggtg gtatagggcg tttggccctg tgactatggc tctggcac(c/t)a
ctagggtcct

In SEQ ID No 1 ist die vollständige cDNA-Sequenz aufgelistet.

Bezogen auf die genomische Sequenz des GBN3 Lokus, wie er von Ansari-Lari und Mitarbeitern beschrieben wurde (Ansari-Lari, M.A., Muzny,D.M., Lu,J., Lu,F., Lilley,C.E., Spanos,S., Malley,T. und Gibbs,R.A., A gene-rich cluster between the CD4 and triosephosphate isomerase genes at human chromosome 12p13.Genome Res. 6 (4), 314-326 (1996)), ist dieser Polymorphismus wie folgt lokalisiert (C59308T):

59281 TTGGCCCTGT GACTATGGCT CTGGCAC(C/T)AC TAGGGTCCTG
GCCCTCTTCT TATTCATGCT

In SEQ ID No 2 ist die vollständige genomische Sequenz aufgelistet.

Der Nachweis dieses Polymorphismus erfolgt durch dem Fachmann geläufige Methoden wie spezifische Hybridisierung, Sequenzierung, PCR- Reaktion mit anschließender Restriktionsanalyse, DNA - Chip- Technologie, single strand conformation polymorphism etc.. In einem beispielhaften Versuch erfolgte der Nachweis durch Amplifikation des entsprechenden Genabschnitts und anschließende Analyse des Restriktions-Fragmentlängen-Polymorphismus, bei dem die Restriktionsenzyme *Ban*I, *Bsh*NI, *Eco*64I oder deren Isoschizomere verwendet werden.

Als Ergebnis findet sich eine große Übereinstimmung zwischen dem Genotyp an Positionen 825 und 1429:

| Genotyp an Position 825 der GNB3 cDNA | Genotyp TT bei Position 1429 der cDNA | Genotyp TC bei Position 1429 der cDNA | Genotyp CC bei Position 1429 der cDNA |
|---|---|---|---|
| TT 119 | 111 (93,3 %) | 8 (6,7%) | 0 |
| TC 116 | 3 (2,6 %) | 103 (88,8 %) | 10 (8,6 %) |
| CC 124 | 0 (0 %) | 2 (1,6 %) | 122 (98,4 %) |

Die neue Nukleinsäuresequenz kann zur Herstellung von Antisense-Arzneimitteln zur Therapie oder Prävention von Krankheiten dienen, wobei eine zu dieser Nukleinsäuresequenz komplemetäre Nukleinsäuresequenz zur Herstellung des Antisense-Arzneimittels eingesetzt wird. Die Patienten können dabei z.B. mit Antisense-Oligonukleotiden oder Vektoren zur Verhinderung der Transkription oder Translation der Gβ3-Untereinheit behandelt werden.

Gegenstand der Erfindung ist ferner die Verwendung von von β3-Untereinheiten der G-Proteine zur Ermittlung des Risikos, an einer Krankheit, die mit einer G-Protein-Fehlsteuerung assoziiert ist, zu erkranken.

Obwohl die Gβ3s- Spleißvariante, die den Polymorphismus C1429T aufweist bzw. auf den Polymorphismus C825T zurückzuführen ist, in Kombination mit den G-Protein-Untereinheiten Gαi2 und Gγ5 ein funktionelles Heterotrimer bilden kann, war es unklar, auf welche Weise Gβ3s zu einer gesteigerten Aktivierbarkeit von G-Proteinen führt.

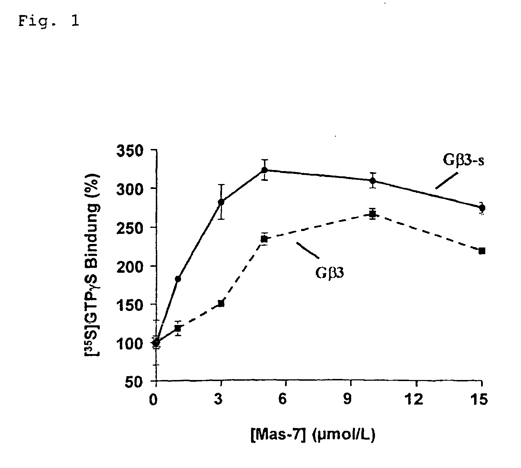

Der Gegenstand der vorliegenden Erfindung beruht auf der Erkenntnis, daß die Untereinheit Gβ3s zu einer gesteigerten Aktivierbarkeit von G-Proteinen führt. Der Nachweis der gesteigerten Aktivierbarkeit von G-Proteinen erfolgte über die Transfektion der entsprechenden cDNAs und Expression von Gβ3 und Gβ3s im dem Fachmann wohlbekannten COS-7-Transfektionssystem. Hier zeigte sich, daß die Aktivierbarkeit von G-Proteinen nach Transfektion von Gβ3s gegenüber Gβ3 deutlich gesteigert ist (Fig.1). Zur Quantifizierung der Aktivierbarkeit von G-Proteinen wurde hier der Einbau von radioaktiv markiertem [35S]GTPγS in G-Protein α-Untereinheiten nach Stimulation mit dem Peptid Mastoparan-7 (Mas-7) gemessen.

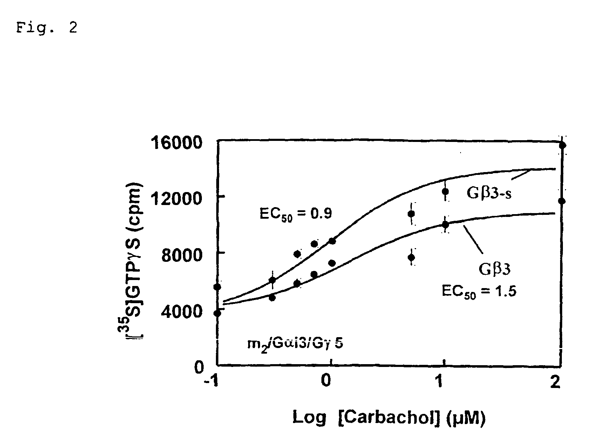

Ähnliche Ergebnisse lassen sich im Sf9-Insektenzellen-Expressionssystem erzielen (Fig.2). Hier wird der m2-muskarinerge Rezeptor zusammen mit den G-Protein-Untereinheiten Gαi3 und Gγ5 und entweder Gβ3 oder Gβ3-s im Sf9-System exprimiert. Nach Stimulation mit dem Agonisten Carbachol werden in Gegenwart von Gβ3-s eine gesteigerte Potenz und eine gesteigerte Effizienz des Agonisten Carbachol bezogen auf die Stimulierbarkeit von G-Proteinen beobachtet.

Diese Versuche zeigen, daß die Spleißvariante Gβ3-s für eine gesteigerte Aktivierung von G-Proteinen ursächlich verantwortlich ist. Ferner beweisen diese Versuche, daß das Protein sich für eine Gentherapie für Krankheiten, die mit einer solchen G-Protein-Fehlsteuerung assoziiert sind, im Sinne der Erzielung einer gesteigerten zellulären Reaktivität eignet.

Unter Krankheiten, die mit einer G-Protein-Fehlsteuerung assoziiert sind, sind solche Erkrankungen zu verstehen, bei denen das G-Protein in der Signaltransduktion involviert ist und seine Funktion nicht in physiologischer Weise erfüllt. Die Fehlsteuerung kann eine Reihe von Ursachen haben, beispielsweise eine Veränderung im Strukturgen oder eine veränderte Genexpression. Die vorliegende Erfindung betrifft Krankheiten, die mit dem oben beschriebenen GNB3-825T-Allel bzw. GNB3-1429T-Allel assoziiert sind. Dazu zählen Diabetes mellitus (Typ-2), Übergewicht und Adipositas, koronare Herzkrankheit, Immunerkrankungen infolge einer verstärkten Funktion des Immunsystems, und Risikoschwangerschaften mit der Gefahr einer vorzeitigen Geburt (Frühgeburt).

Die folgenden Beispiele beziehen sich zwar auf Untersuchungen mit dem GNB3-825T-Allel. Aufgrund der großen Übereinstimmung (Kopplungsgleichgewicht) zwischen dem Genotyp an Positionen 825 und 1429 sind diese Beispiele und die aus den Versuchsergebnissen gezogenen Schlußfolgerungen für das GNB3-1429T-Allel ebenso gültig.

### 1. Vorhersage des Diabetes mellitus (Typ-2)

Der Typ-2-Diabetes (Synonyme: Altersdiabetes, nicht-Insulinpflichtiger Diabetes) ist eine schwerwiegende Erkrankung mit hoher kardiovaskulärer Morbidität und Mortalität. Genetische Einflüsse und Übergewicht tragen wesentlich zur Pathogenese bei. Der Typ-2-Diabetes beginnt häufig als Insulinresistenz, welche zunächst durch eine gesteigerte Insulinsekretion kompensiert wird, so daß die betroffenen Individuen symptomlos (euglykämisch) bleiben. Erst wenn die gesteigerte Insulinsekretion nicht mehr aufrecht erhalten werden kann, kommt es zum Diabetes mit gesteigerten Blutzuckerspiegeln. Auf zellulärer Ebene können Veränderungen in Komponenten der Insulinsignaltransduktion, z.B. bei Insulin- Rezeptor-Substrat 1 (IRS-1), PI-3-Kinasen, Proteinkinasen, etc. eine Insulinresistenz bewirken. Aber auch nach einer Zellstimulation mit Agonisten (z.B. Angiotensin II), die G-Protein-gekoppelte Rezeptoren aktivieren, kann eine zelluläre Insulinresistenz herbeigeführt werden. Die Wirkung einer nachfolgenden Stimulation mit Insulin ist dann deutlich vermindert (Polonsky, K.S., Sturis, J., and Bell, G.I. Non-insulin-dependent diabetes mellitus - A genetically programmed failure of the beta cell to compensate for insulin resistance. N.Engl.J.Med. 334:777-783, 1996; O'Doherty, R., Stein, D., and Foley, J. Insulin resistance. Diabetologia 40 Suppl 3:B10-5:B10-5, 1997; Kahn, C.R., Vicent, D., and Doria, A. Genetics of non-insulin-dependent (type-II) diabetes mellitus. Annu.Rev.Med. 47:509-531, 1996; Hansen, T., Andersen, C.B., Echwald, S.M., Urhammer, S.A., Clausen, J.O., Vestergaard, H., Owens, D., Hansen, L., and Pedersen, O. Identification of a common amino acid polymorphism in the p85alpha regulatory subunit of phosphatidylinositol 3-kinase: effects on glucose disappearance constant, glucose effectiveness, and the insulin sensitivity index. Diabetes 46(3):494-501, 1997; Folli, F., Kahn, C.R., Hansen, H., Bouchie, J.L., and Feener, E.P. Angiotens in II inhibits insulin signaling in aortic smooth muscle cells at multiple levels - A potential role for serine phosphorylation in insulin/angiotensin II crosstalk. J.Clin.Invest. 100:2158-2169, 1997; Zhang, Y., Wat, N., Stratton, I.M., Warren-Perry, M.G., Orho, M., Groop, L., and Turner, R.C. UKPDS 19: heterogeneity in NIDDM: separate contributions of IRS-1 and b3-adrenergic receptor mutations to insulin resistance and obesity respectively with no evidence for glycogen synthase gen mutations. Diabetologia 39:1505-1511, 1996; Almind, K., Bjorbaek, C., Vestergaard, H., Hansen, T., Echwald, S., and Pedersen, O. Aminoacid polymorphisms of insulin receptor substrate-1 in non-insulin-dependent diabetes mellitus. Lancet 342:828-832, 1993; Laakso, M., Malkki, M., Kekäläinen, P., Kuusisto, J., and Deeb, S.S. Insulin receptor substrate-1 variants in non-insulin-dependent diabetes. J.Clin.Invest. 94:1141-1146, 1994).

Diese G-Protein-Aktivierung führt zu einer Phosphorylierung von IRS-1 an Serinresiduen, wodurch die durch Insulin induzierte Phosphorylierung an Tyrosinresiduen reduziert wird.

Als Folge davon kommt es zu einer reduzierten Interaktion von IRS-1 mit dem Insulinrezeptor und der PI-3-Kinase, d.h. zu einer verminderten Insulinwirkung. Eine gesteigerte Aktivierbarkeit von G-Proteinen, die durch das GNB3 825T-Allel und die damit verbundene Spleißvariante Gβ3-s in vivo bewirkt wird, verstärkt die Neigung zur Insulinresistenz deutlich.

Es hat sich nun gezeigt, daß bei gleichzeitigem Vorliegen von Mutationen in Komponenten der Insulinsignaltransduktion (IRS1-Gen, 3931A-Variante; Gly971Arg; p85α- regulatorische Untereinheit der PI3-Kinase (1020 G →A; Codon 326 Met →Ile; β3-adrenerger Rezeptor (Trp64Arg); β2-adrenerger Rezeptor (hier insbesondere die Arg16Gly- Variante und die Gln27Glu-Variante); Tumornekrosefaktor α; Leptin oder der Leptinrezeptor), welche zur Insulinresistenz führen, und dem GNB3-825T-Allel, die Neigung zur Insulinresistenz und zum Diabetes drastisch ansteigt. Dieser Zusammenhang eröffnet die Möglichkeit, eine mit dem GNB3-825T-Allel assoziierte Diabetes mellitus vom Typ 2 zu diagnostizieren und eine derart genetisch bedingte Neigung zur Diabetes mellitus vom Typ 2 bei noch gesunden bzw. beschwerdefreien Personen vorherzusagen.

Zum Beweis wurde die DNA von über 700 Patienten mit Typ-2-Diabetes und von 1400 gesunden Kontrollpersonen gewonnen. Die Häufigkeiten des GNB3-825T-Allels und der IRS-1-Gly971Arg-Variante wurden verglichen. Tabelle I zeigt zunächst einen Vergleich der Allelfrequenzen bei Kontrollen und Fällen:

**Tabelle I: Allelfrequenzen bei Kontrollen und Typ-2-Diabetikern**

| | | **Kontrollen** | | | **Diabetiker** | | |
|---|---|---|---|---|---|---|---|
| | | **Alle** | **Männer** | **Frauen** | **Alle** | **Männer** | **Frauen** |
| Total, n | | 1464 | 962 | 502 | 720 | 320 | 400 |
| Genotyp, n (%): | | | | | | | |
| GNB3 | TT | 116 (8) | 83 (9) | 33 (7) | 61 (8) | 32 (10) | 29 (7) |
| | TC | 585 (40) | 360 (37) | 225 (45) | 345 (48) | 161 (50) | 184 (46) |
| | CC | 763 (52) | 519 (54) | 244 (49) | 314 (44) | 127 (40) | 187 (47) |
| | FT | 0.28 | 0.27¹ | 0.29 | 0.32² | 0.35³ | 0.30 |
| IRS1 | AA | 5 (0) | 4 (0) | 1 (0) | 8 (1) | 4 (1) | 4 (1) |
| | AG | 159 (11) | 108 (11) | 51 (10) | 108 (15) | 58 (18) | 50 (13) |
| | GG | 1300 (89) | 850 (88) | 450 (90) | 604 (84) | 258 (81) | 346 (87) |
| | FA | 0.06 | 0.06 | 0.05 | 0.09⁴ | 0.10⁵ | 0.07⁶ |
| Alter (SD) | | 49 (10) | 48 (10) | 50 (9) | 63 (9) | 62 (9) | 64 (9) |
| Alter bei Diagnose (SD) | | | | | 46 (10) | 45 (10) | 47 (10) |
| **BMI, kg / m**^{**2**} **(SD)** | | 26.9 (3.9) | 27.0 (3.4) | 26.3 (4.8) | 28.9 (4.8) | 28.4 (4.4)⁶ | 29.3 (5.0) |
| Nephropathy, n (%) | | | | | 198 (31) | 105 (36) | 93 (28) |
| Hypertension, n (%) | | | | | 464 (67) | 181 (59) | 283 (73) |

| | | | | | | | |
|---|---|---|---|---|---|---|---|
| Werte entsprechen n (%) für Allele und Diabetes-assoziierte Erkrankungen, und Mittelwerte (SD) für kontinuierliche Variablen. ¹, p < 0.02 (χ² = 8.1) versus Kontrollfrauen; ², p < 0.001 (χ² = 14.4) versus alle Kontrollen; ³, p < 0.001 (χ² = 20.0) versus Kontrollmänner; ⁴, p < 0.005 (χ² = 12.9) versus alle Kontrollen; ⁵, p < 0.01 (χ² = 13.2) versus Kontrollmänner; ⁶, p = 0.14 (χ² = 3.1) versus Kontrollfrauen. | | | | | | | |

Die folgenden Tabellen IIa und IIb zeigen das Risiko für Träger des GNB3-825T-Allels bzw. Träger des IRS1-3931A-Allels (einfache Effekte, Tabelle II.1) bzw. für Träger beider Allele (Kombinierte Effekte, Tabelle II.2), an Typ-2-Diabetes zu erkranken. Das Risiko ist hier als altersadjustierte Odds Ratio ausgedrückt, wobei die Odds Ratio für Fall-Kontroll-Studien etwa dem relativen Risiko bei prospektiven Studien entspricht.

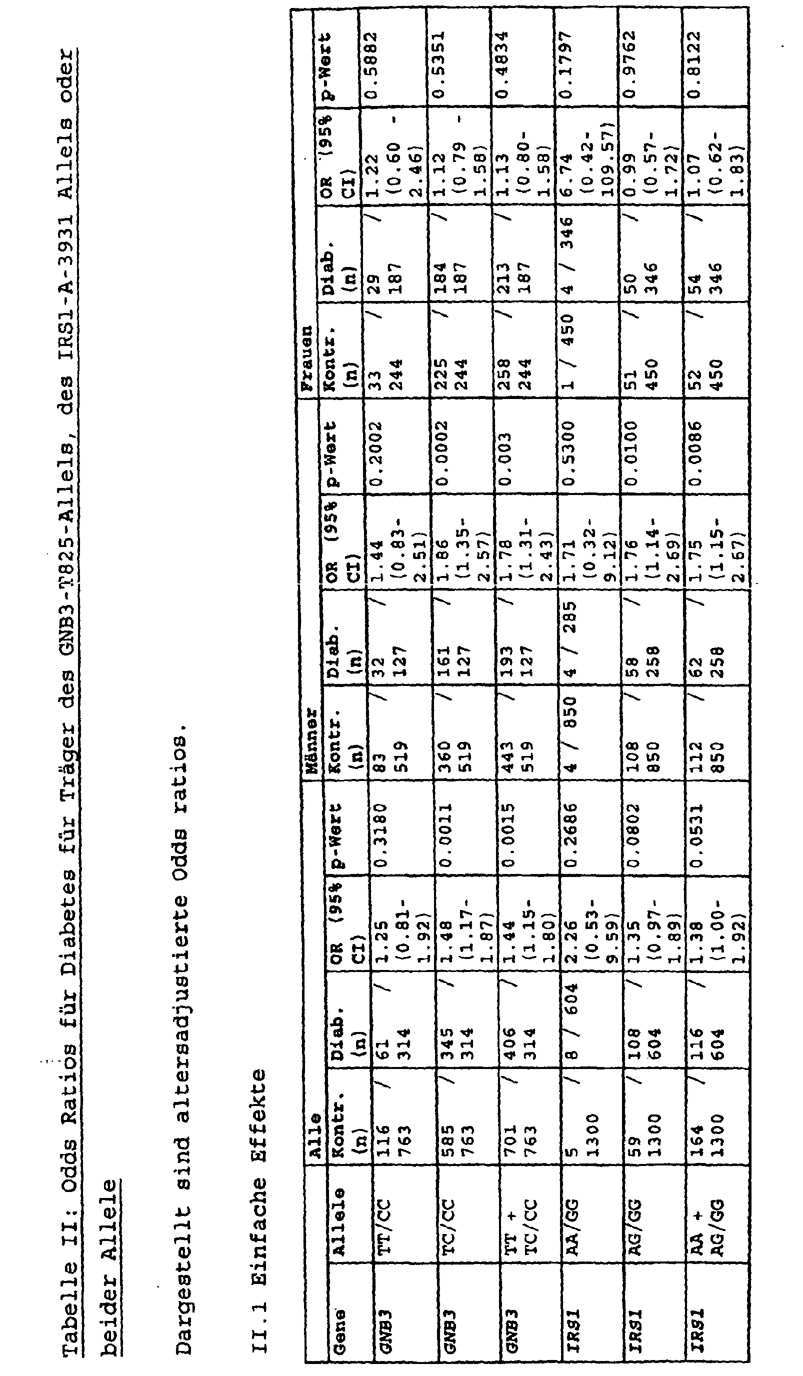

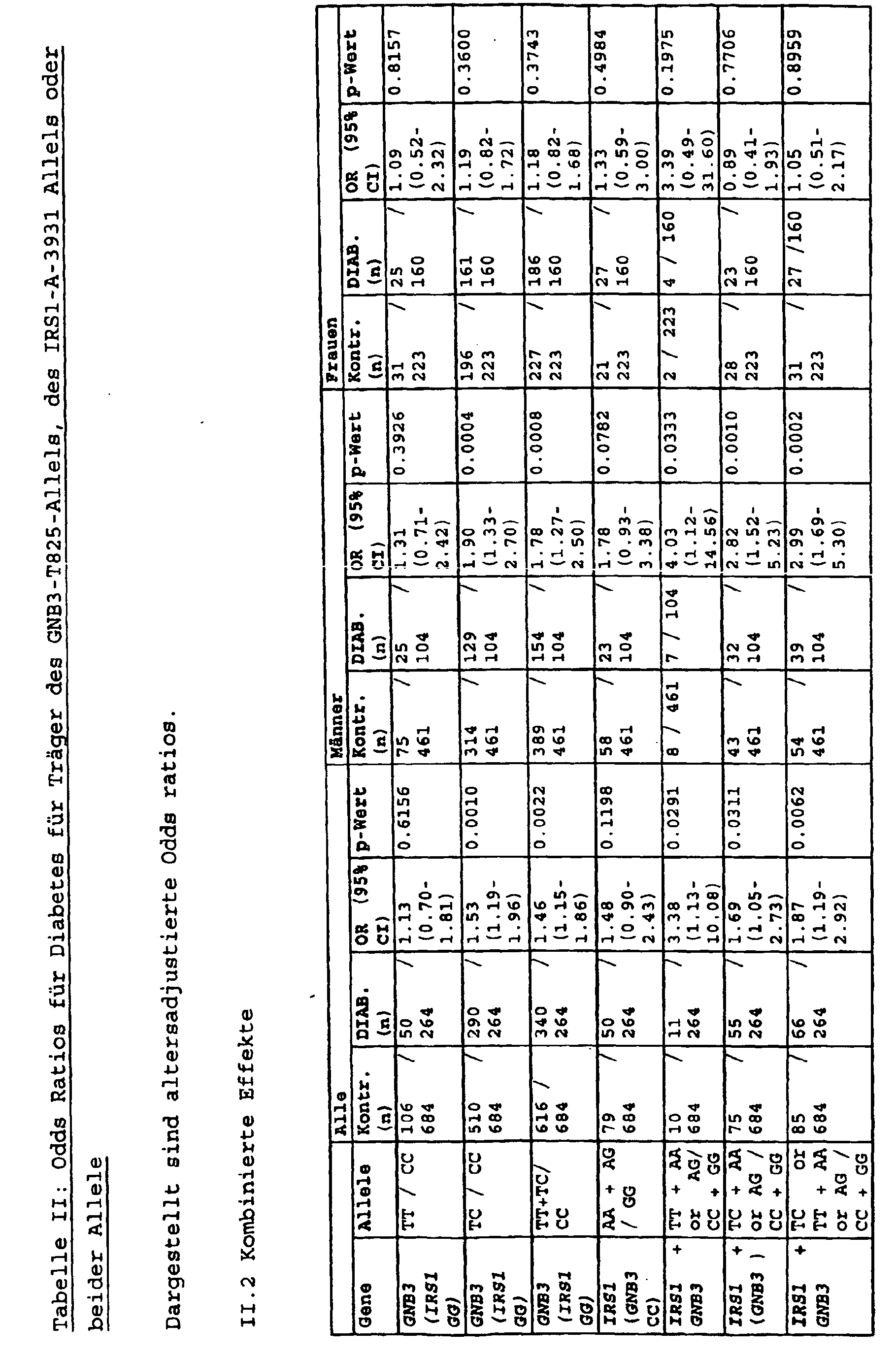

Man erkennt eine deutliche Risikosteigerung für den Typ-2-Diabetes für Träger des GNB3-825T-Allels bzw. Träger des IRS1-3931A-Allels (Tabellen II.1 und II.2). Eine drastische Risikosteigerung findet man bei Individuen, bei denen beide Gene verändert sind. Somit kann der Nachweis des GNB3-825T-Allels dazu dienen, bei den betroffenen Personen die Neigung für eine Erkrankung an Typ-2-Diabetes festzustellen bzw. eine genetisch bedingte Ursache einer bereits aufgetretenen Erkrankung zu ermitteln.

### 2. Vorhersage von Adipositas/Übergewicht

Die Regulation des Body Mass Index (BMI), ein Maß für das Verhältnis von Körpergewicht zu Körpergröße, wird durch viele Gene bestimmt. Übergewicht ist zu etwa 40% genetisch bedingt, wird aber auch durch überhöhte Kalorienzufuhr bei bestehendem Bewegungsmangel verursacht. Die Gβ3-s Spleißvariante ist mit dem zellulären Phänotyp eines gesteigerten Zellwachstums verbunden. Damit ist es auch möglich, daß das GNB3-825T-Allel zu einem gesteigerten Körperwachstum, u.a. Übergewicht, prädisponiert, und epi- und hypostatische Effekte ausüben kann. Dabei kann ebenso wie beim Diabetes mellitus (Typ 2) ein Zusammenhang bestehen mit Veränderungen im IRS1-Gen (3931A-Variante; Gly971Arg), im Gen, das für den β3-adrenergen Rezeptor kodiert (Trp64Arg-Variante) und im Gen, das für den β2-adrenergen Rezeptor kodiert, hier insbesondere die Arg16Gly- Variante und die Gln27Glu-Variante.

Untersuchungen an transgenen Mäusen zeigten, daß das Fehlen des Gens, welches für das IRS-1-Protein kodiert, im Gegensatz dazu zu einer starken Verzögerung des Körperwachstums führt (Tamemoto, H., Kadowaki, T., Tobe, K., Yagi, T., Sakura, H., Hayakawa, T., Terauchi, Y., Ueki, K., Kaburagi, Y., Satoh, S., Sekihara, H., Yoshioka, S., Horikoshi, H., Furuta, Y., Ikawa, Y., Kasuga, M., Yazaki, Y., and Aizawa, S. Insulin resistance and growth retardation in mice lacking insulin receptor substrate-1. Nature 372:182-186, 1994).

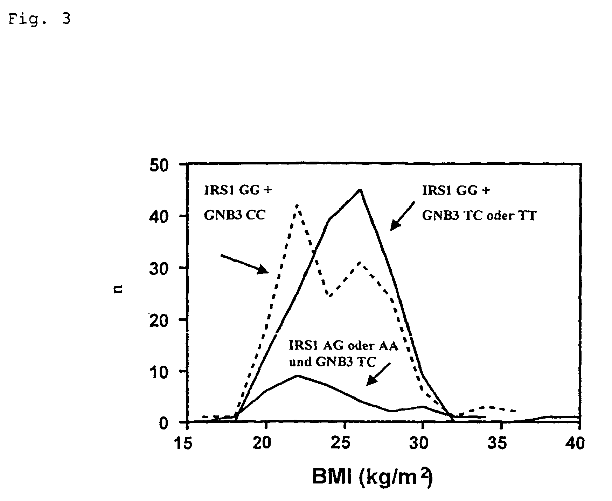

Um die Korrelation von Adipositas mit dem Vorliegen des GNB3-825-Nukleotids und der Status des IRS1-Gens bezüglich des Vorliegens der Gly971Arg-Variante zu überprüfen, wurde der BMI bei 20-30-jährigen gesunden Männern, bei denen gleichzeitig der Status des GNB3-825-Nukleotids und der Status des IRS1-Gens bezüglich des Vorliegens der Gly971Arg-Variante untersucht wurde, gemessen. Das Ergebnis ist in Figur 3 dargestellt.

Fig. 3 zeigt die Häufigkeitsverteilung der gemessenen Werte für den BMI. Bei Vorliegen des häufigeren IRS1-"GG"-Genotyps läßt sich mit dem GNB3-825T-Allel im Vergleich zum GNB3-C825-Allel eine signifikante Tendenz zu erhöhtem BMI nachweisen. Die Odds Ratio für das 75%-Quartil gegenüber dem 25 % Quartil beträgt 2,5. Dagegen wird der Neigung zu gesteigertem BMI bei Trägern des GNB3-825T-Allels bei gleichzeitiger Anwesenheit des IRS1-3931A-Allels (Gly971Arg-Variante) deutlich entgegengewirkt. Dies illustriert den interaktiven Effekt von GNB3 und IRS1 auf den BMI. Somit läßt sich das Vorliegen des GNB3-825T-Allel zuverlässig mit Adipositas korrelieren. Damit ist es möglich, eine Neigung zu Adipositas bei Trägern dieses Allels, insbesondere solchen, denen gleichzeitig das IRS1-3931A-Allel (Gly971Arg-Variante) fehlt, vorherzusagen.

825T-Allelträger weisen, wie z.B. in der DE 196 19 362 A1 beschrieben, ein erhöhtes Risiko auf, an einer Hypertonie zu erkranken. Da Übergewicht und Adipositas in sehr starkem Maße für kardiovaskuläre Erkrankungen prädisponieren, wurde untersucht, ob junge Personen mit normalem Blutdruck, die ein 825T-Allel tragen, bereits ein erhöhtes Risiko für Übergewicht und Adipositas aufweisen. Dazu wurde bei 277 jungen, normotensiven Männern die Körpergröße und das Körpergewicht bestimmt und der Blutdruck gemessen. Übergewicht wird als ein BMI ≥ 25,0 kg/m² definiert und Adipositas als ≥ 27,0 kg/m². Es besteht ein deutlicher Zusammenhang zwischen BMI und Blutdruckwerten. Die Frequenz des 825T-Allels steigt über vom 1. zum 4. BMI-Quartil linear an. Für homozygote 825T-Allelträger lassen sich die folgenden Risiken (odds ratios; OR) berechnen
a) BMI ≥ 25,0 kg/m² *versus* BMI < 25 kg/m² (Übergewicht versus Normalgewicht):
   OR TT/CC = 2,5 (1,1 - 6,1; p = 0,03); OR TC/CC 1,5 (0,8 - 2,6; p = 0,2)
b) BMI > 27 kg/m² versus BMI < 25 kg/m² (Adipositas versus Normalgewicht) :
   OR TT/CC = 5,0 (1,4 - 18,3; p = 0,0083); OR TC/CC = 2,2 (0,8 - 6,3; p = 0,13).

Somit ergibt sich ein eindeutiger Zusammenhang zwischen Vorhandensein eines 825T-Allels und der Neigung zu Übergewicht und Adipositas. Dies erklärt unter anderem zum Teil das erhöhte Risiko von 825T-Allelträgern für Hypercholesterämie, Diabetes, Hypertonie und koronare Herzkrankheit/Myokardinfarkt.

**GNB3 825T - Allel, BMI und Blutdruckwerte**

| | | BMI Quartile | | | | |
|---|---|---|---|---|---|---|
| *GNB3* | Alle | 1 < 21.7 | 2 21.7 - 23.4 | 3 23.4- 25.0 | 4 ≥ 25.0 | > 27 kg/m2 |
| TT | 28 (10) | 2 (3) | 6 (8) | 9 (13) | 11 (16) | 5 (23) |
| TC | 121 (44) | 27 (39) | 27 (40) | 34 (49) | 33 (47) | 11 (50) |
| CC | 128 (46) | 40 (58) | 36 (52) | 26 (38) | 26 (37) | 6 (27) |
| Σ | 277 | 69 | 69 | 69 | 70 | 22 |
| fT (%) | 31.9 | 22.5 | 28.3 | 37.7 | 39.3 | 47.7 |
| Alter (Jahre) | 25.6 (3.4) | 24.8 (3.6) | 25.6 (3.2) | 25.3 (3.4) | 26.4 (2.9) | 26.5 (2.9) |
| Größe (cm) | 180.4 (7.3) | 180.8 (6.4) | 180.5 (7.4) | 180.7 (7.1) | 179.6 (7.4) | 181.0 (5.9) |
| Gewicht (kg) | 76.5 (9.8) | 67.4 (5.2) | 73.1 (6.3) | 78.9 (7.1) | 86.2 (8.7) | 93.5 (6.9) |
| BP syst (mm Hg) | 129.8 (11.1) | 126.3 (9.1) | 130.2 (9.4) | 130 (11.8) | 133 (12.9) | 135.9 (10.7) |
| BP diast (mm Hg) | 79.1 (7.9) | 75.8 (7.9) | 79.4 (5.5) | 79.7 (7.3) | 81.9 (9.2) | 84.3 (9.8) |

Genotypen sind als n (%) angegeben und kontinuierliche Variablen als Mittelwerte (Standardabweichung); fT = 825T Allelfrequenz; BMI (body mass index) ist als kg/m² angegeben. BP syst = systolischer Blutdruck; BP diast = diastolischer Blutdruck.

### 3. Vorhersage von koronarer Herzkrankheit und Atherosklerose

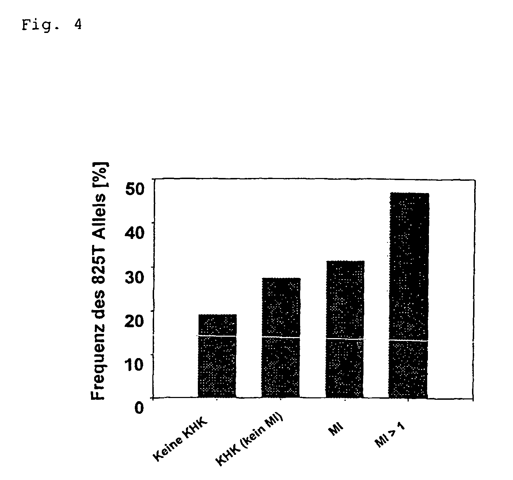

Es ist bereits bekannt, daß koronare Herzkrankheit mit einer G-Protein-Fehlsteuerung assoziiert sein können. Um einen Zusammenhang zwischen dem Auftreten einer koronaren Herzkrankheit und dem Vorhandensein des GNB3-825T-Allels zu überprüfen, wurden Patienten mit angiographisch ausgeschlossener koronarer Herzkrankheit, mit koronarer Herzkrankheit (ohne Myokardinfarkt), mit einem Myokardinfarkt und mit mehr als einem Myokardinfarkt auf das Vorhandensein dieses Alles überprüft. Das Ergebnis ist in Fig. 4 dargestellt.

Fig. 4 zeigt die Frequenz des GNB3-825T-Allels bei Patienten mit angiographisch ausgeschlossener koronarer Herzkrankheit (KHK), mit KHK (ohne Myokardinfarkt; MI), mit einem Myokardinfarkt (MI) und mit mehr als einem Myokardinfarkt.

Man erkennt einen deutlichen Anstieg der Frequenz des GNB3-825T-Allels bei KHK und MI. Das Risiko für KHK und MI wird durch das GNB3-825T-Allel gegenüber Kontrollen ohne KHK etwa verdoppelt.

Patienten mit Mutation im IRS-1 Protein (3931A-Allel; Gly971Arg-Variante) erfahren jedoch eine deutliche Risikoreduktion um bis zu 50 % bei Vorliegen des GNB3-C825- oder des GNB3-825T-Allels. Diese Veränderung im IRS-1-Protein übt also hypostatische Effekte aus, d.h. diese Variante schützt vor koronarer Herzkrankheit.

Vergleicht man Patienten mit KHK mit Individuen mit koronarangiographisch ausgeschlossener KHK, so ergeben sich die folgenden Odds Ratios:

| | | | |
|---|---|---|---|
| KHK positiv | KHK negativ | OR | P - Wert |
| GNB3 + IRS1-Status | GNB3 + IRS1-Status | | |
| TT/TC + AG/AA | TT/TC + GG | 0.94 | Nicht signifikant |
| TT/TC + GG | CC + GG | 1,4 | 0,003 |
| TT/TC + GG | CC + AG/AA | 2,8 | 0,002 |

Damit ist eine Korrelation zwischen dem Vorliegen des GNB3-825T-Allels mit dem Auftreten koronarer Herzkrankheit belegt. Somit ist es möglich, eine Neigung zu koronarer Herzkrankheit bei Trägern dieses Allels, insbesondere solchen, denen gleichzeitig das IRS1-3931A-Allel (Gly971Arg-Variante) fehlt, vorherzusagen.

Ein besonderer Anwendungsbereich ist die Vorhersage einer koronaren Herzkrankheit, aber auch allgemein des kardiovaskulären Risikos (Bluthochdruck, usw.) bei Frauen, mit dem Ziel, diese einer gezielten, postmenopausalen Hormontherapie mit weiblichen Sexualhormonen zuzuführen, um das kardiovaskuläre Risiko zu vermindern.

Ein weiterer Anwendungbereich ist die Vorhersage eines erhöhten Risikos für Myokardinfarkte und plötzlichen Herztod. Dies hängt u.a. damit zusammen, daß G-Proteine auch Ionenkanäle steuern. Genauer gesagt, steuern die Gα-und Gβγ-Untereinheiten von G-Proteinen die Funktion vielfältiger Ionenkanäle, z.B. von Na⁺-Kanälen, Ca²⁺⁻Kanälen und K⁺-Kanälen. Eine genau abgestimmte Regulation solcher Ionenkanäle ist für alle elektrisch erregbaren Gewebe von großer Wichtigkeit, insbesondere für das Herz (De Waard, M., Liu, H., Walker, D., Scott, V.E., Gurnett, C.A., and Campbell, K.P. Direct binding of G-protein βγ complex to voltage-dependent calcium channels. Nature 385(6615):446-450, 1997; Ma, J.Y., Catterall, W.A., and Scheuer, T. Persistent sodium currents through brain sodium channels induced by G protein βγ subunits. Neuron 19(2):443-452, 1997; Kofuji, P., Davidson, N., and Lester, H.A. Evidence that neuronal G-protein-gated inwardly rectifiying K⁺ channels are activated by Gβγ subunits and function as heteromultimers. Proc.Natl.Acad.Sci.USA 92:6542-6546, 1995; Krapivinsky, G., Krapivinsky, L., Wickman, K., and Clapham, D.E. Gβγ binds directly to the G protein-gated K⁺ channel, I_{KACh}. J.Biol.Chem. 270:29059-29062, 1995).

Es hat sich gezeigt, daß Personen, die das GNB3-T825-Allel tragen, eine verstärkte Aktivität myokardialer K⁺⁻Kanäle zeigen. Dies führt zu einer beschleunigten Repolarisation der Herzmuskelzelle, und damit zu einer verkürzten Refraktärzeit. Diese Personen unterliegen damit einem erhöhten Risiko für Herzrhythmusstörungen, insbesondere ventrikuläre Tachykardien, Extrasystolen, Kammerflattern und Kammerflimmern. Sie tragen ein verstärktes Risiko für einen plötzlichen Herztod auch im Rahmen eines akuten Myokardinfarkts.

Schließlich zeigen Träger des GNB3-825T-Allels bereits im Alter von 20 - 30 Jahren deutliche Veränderungen der Eigenschaften von Blutgefäßen. Besonders auffällig sind eine erhöhte Pulswellengeschwindigkeit, ein gesteigertes Schlagvolumen des Herzens und ein erhöhter Pulsdruck. Diese Phänomene sind Ausdruck einer früh einsetzenden Neigung zu einer erhöhten Steife der Blutgefäße (verminderte Compliance) als Indikator für eine Atherosklerose. Die Genotypisierung zur Feststellung des GNB3-C825T-Allelstatus ist damit geeignet, ein erhöhtes Risiko für die Atherosklerose festzustellen.

### 4. Vorhersage einer erhöhten Cholesterinkonzentration im Blut

Es ist allgemein bekannt, daß Menschen mit erhöhter Konzentration des Gesamtcholesterin im Blut ein erhöhtes Risiko für koronare Herzkrankheit und Herzinfarkt zuzuordnen ist. Es wurden 232 Personen im Alter von 18 - 40 Jahren bezüglich des C825T - Polymorphismus in GNB3 genotypisiert und das Gesamtcholesterin im Serum wurde mittels Standardmethodik quantifiziert. Nachfolgend wurden die gemessenen Cholesterinkonzentrationen (mg/dl) in Quartile aufgeteilt, und der Genotyp am GNB3 - Locus wurde den Quartilen zugeordnet. Die niedrigste Frequenz des 825T-Allels findet sich mit 23,3 % im 1. Quartil, während die Frequenz des 825T-Allels in den Quartilen 2-4 deutlich höher liegt.

| | Cholesterinkonzentration | | | |
|---|---|---|---|---|
| | 1. Quartil - 163 mg/dl - | 2. Quartil 181 mg/dl - | 3. Quartil 212 mg /dl | 4. Quartil > 212 mg/dl |
| TT | 4 (9) | 4 (8) | 12 (17) | 7 (12) |
| TC | 13 (29) | 21 (44) | 32 (46) | 28 (47) |
| CC | 28 (62) | 23 (48) | 26 (37) | 24 (41) |
| ft | 23,3 % | 30,2 % | 40,0 % | 35,6 % |

### Die Zahlen entsprechen n (%)

Vergleicht man die Genotypverteilung oberhalb des Medianwertes ( > 181 mg/dl; TT = 19; TC = 60; CC = 50; Frequenz des 825T-Allels: 38 %) mit der unterhalb des Medianwertes ( ≤ 181 mg/dl; TT = 8; TC = 34; CC = 51; Frequenz des 825T-Allels:26,9 %), so errechnen sich die folgenden Risiken für Cholesterinwerte im Bereich oberhalb des Medians :

Odds ratio TT/CC = 2,4 (p = 0,053); odds ratio TC/CC = 1,8 (p < 0.05)

Damit ist das 825T- Allel mit einem erhöhten Risiko für eine Hypercholesterinämie verbunden.

Eine Genotypisierung am GNB3-Locus bietet also die Möglichkeit zur Feststellung eines erhöhten Risikos für eine Hypercholesterinämie und mit dem Ziel, betroffene Personen mit Pharmaka zu behandeln, die das erhöhte Cholesterin senken können. Dazu gehören insbesondere Hemmstoffe des Enzyms 3-Hydroxy-3-methyl-glutaryl-Coenzym A-Reduktase (HMG-CoA-Reduktase), z.B. Simvastatin, Pravastatin, Fluvastatin, Lovastatin, Atorvastatin und weitere sog. "Statine". Dazu gehören auch β-Sitosterin, Sitostanol-Ester (auch in Lebensmitteln), Fibrate und weitere Substanzen, die das Cholesterin senken.

Die genannten Pharmaka wirken hierbei auch als G-Protein Hemmer und lassen sich somit bei mit einer G-Protein-Fehlsteuerung assoziierten Krankheiten therapeutisch einsetzen.

### 5. Vorhersage einer verstärkten Funktion des Immunsytems

G-Proteine und G-Protein-gekoppelte Rezeptoren finden sich auch in allen Zellen des Immunsystems, insbesondere auch in Leukozyten. Chemotaxis von Zellen wird vorwiegend durch βγ-Untereinheiten heterotrimerer G-Proteine vermittelt. Damit sollte auch das GNB3-825T-Allel zu einer gesteigerten Reaktionsfähigkeit des Immunsystems, insbesondere zu einer verstärkten Immunabwehr führen.

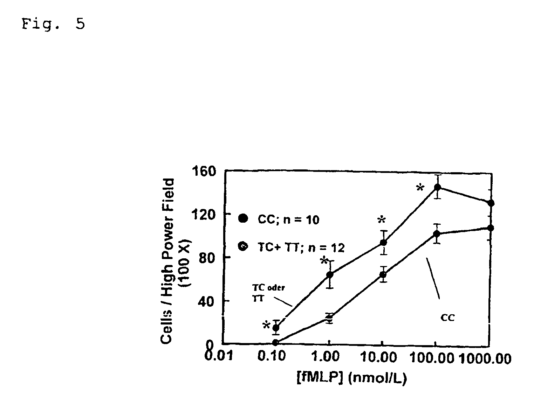

In der Tat weisen neutrophile Granulozyten von Trägern des GNB3-825T-Allels eine verstärkte Chemotaxis gegenüber dem Peptid fMLP auf (Fig.5). fMLP ist ein Peptid, welches für eine Vielzahl von bakteriellen Peptiden repräsentativ ist und chemotaktische Reaktionen stimuliert. Es dient daher als Testsystem für die Messung chemotaktischer Reaktionen von Zellen, welches dem Fachmann wohlbekannt ist. Der fMLP-Rezeptor aktiviert bekanntlich Pertussistoxin-sensitive G-Proteine. Die Feststellung, daß Granulozyten von Trägern des GNB3-825T-Allels eine verstärkte fMLP-stimulierte Chemotaxis zeigen, steht in Einklang mit der Tatsache, daß die Chemotaxis von βγ-Untereinheiten vermittelt wird.

Dieses Phänomen läßt sich auch in anderen Leukozyten, z.B. Lymphozyten, nachweisen. Somit besteht eine Korrelation zwischen dem GNB3-825T- Allel und einer gesteigerten Chemotaxis von Zellen des Immunsystems, z.B. von neutrophilen Granulozyten, T- Lymphozyten, siehe auch 6., oder B-Lymphozyten.

Ferner beobachtet man bei Trägern des GNB3-825T-Allels eine verstärkte Proliferationsneigung von Zellen des Immunsystems, besonders auch nach Impfungen.

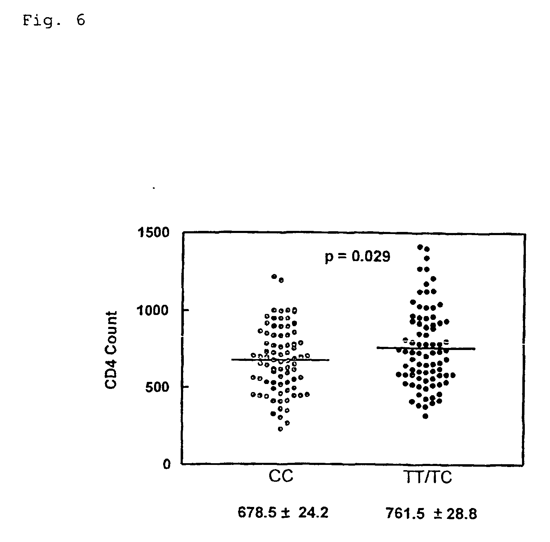

Gesunde Träger des GNB3-825T-Allels zeigen eine erhöhte Anzahl von Leukozyten und von CD4-positiven T-Lymphozyten (absolut und prozentual) mit gesteigertem CD4/CD8 - Quotienten. Fig.6 zeigt dies für die erhöhte Zahl von CD4 - Lymphozyten. Umgekehrt zeigen Träger des GNB3-825T-Allels zeigen auch eine verstärkte Neigung, nach einer HIV-Infektion an AIDS zu erkranken, siehe auch 7., auch im Zusammenhang mit dem Nachweis der oben beschriebenen Genveränderung in Chemokinrezeptoren, insbesondere einer Δ32- Deletion im CCR5- Rezeptor oder im Bereich des Promotors dieses Gens.

Schließlich beobachtet man bei betroffenen Personen eine verstärkte Freisetzung von immunmodulatorischen Substanzen, Hormonen und anderen Substanzen aus Leukozyten (Zytokine, Interleukine, Wachstumsfaktoren, Antikörper, gefäßwirksame Substanzen). In diesem Zusammenhang resultiert auch eine verstärkte Immunabwehr nach Transplantation von Organen oder Geweben (Niere, Herz, Knochenmark, Lunge, Haut, Leber etc.) mit der Gefahr der Transplantatabstossung. Außerdem folgt daraus eine verstärkte Neigung zu Autoimmunerkrankungen (Rheuma, Colitis ulcerosa, Morbus Crohn) und zu allergischen Erkrankungen, z.B. der Haut, der Atemwege oder anderer Organe (z.B. Neurodermitis, Heuschnupfen, Asthma bronchiale). Dies beobachtet man auch in Kombination mit dem Nachweis anderer Genveränderungen, z.B. im β2-adrenergen Rezeptor, hier insbesondere die Argl6Gly-Variante und die Gln27Glu-Variante.

### 6. Vorhersage einer gesteigerten Funktion von T-Lymphozyten

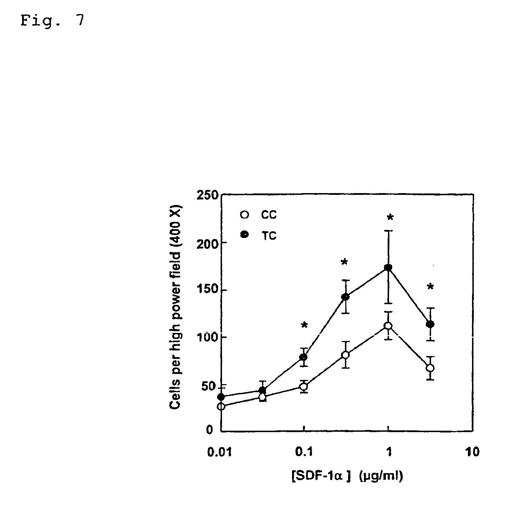

T-Lymphozyten spielen eine wichtige Rolle im menschlichen Immunsystem und vermitteln dort die zelluläre Immunantwort. Eine gesteigerte Aktivierbarkeit von T-Lymphozyten hat unter anderem zur Folge, wie bereits erwähnt, daß transplantierte Organe (Niere, Leber, Herz, Lunge, Pankreas u.a.) einer verstärkten immunologischen Attacke unterliegen. In Fig. 7 ist als ein Beispiel die Chemotaxis menschlicher T-Lymphozyten nach Stimulation mit Stromal Cell-derived Factor 1α (SDF 1α) dargestellt. Man erkennt eine deutlich gesteigerte chemotaktische Antwort der Zellen von 825T - Allelträgern im Vergleich zu Zellen von homozygoten C825-Allelträgern.

In ähnlicher Weise antworten T-Lymphozyten von 825T-Allelträgern verstärkt nach Stimulation mit anderen Chemokinen, z.B. RANTES. Dieses Verhalten erklärt sich daraus, daß die chemotaktische Antwort wesentlich von betagamma - Untereinheiten heterotrimerer G- Proteine gesteuert wird (Arai, H., Tsou, C.L., and Charo, I.F. Chemotaxis in a lymphocyte cell line transfected with C-C chemokine receptor 2B: Evidence that directed migration is mediated by betagamma dimers released by activation of Galphai-coupled receptors. *Proc.Natl.Acad.Sci.U.S.A*. 94(26) :14495-14499, 1997).

Die verstärkte Aktivierung von T-Lymphozyten von 825T-Allelträgern äußert sich auch in einer gesteigerten Proliferation dieser Zellen im Vergleich zu T-Lymphozyten von homozygoten C825 Allelträgern.

Somit läßt sich insgesamt vorhersagen, daß die T-Lymphozyten von 825T-Allelträgern auf geeignete Stimulation stärker reagieren, was sich in einer gesteigerten Proliferation und Chemotaxis äußert. Dieses Verhalten manifestiert sich in Form einer gesteigerten zellulären Immunabwehr, was insbesondere bei Erkrankungen und operativen Eingriffen relevant ist, bei denen eine gesteigerte zelluläre Immunabwehr vorliegt. Zu nennen ist hier besonders die immunologische Attacke von transplantierten Organen (Niere, Leber, Pankreas, Knochenmark, Herz, etc.). Es läßt sich damit vorhersagen, daß 825T-Allelträger vermehrt dazu neigen, gegen solche transplantierten Organe eine akute oder chronische Abstossungsreaktion zu entwickeln. Diese Abstossungsreaktion wird weiter verstärkt, falls die transplantierten Organe von einem Spender stammen, der selbst Träger eines 825T-Allels ist. Dies erklärt sich damit, daß Organe und Gewebe solcher Spender auf die gesteigerte immunologische Attacke durch Zellen des Empfängers bei Vorhandensein eines 825T-Allels verstärkt reagieren. Ferner findet sich eine verstärkte Reaktion bei akuten oder chronischen Virusinfektionen.

### 7. Vorhersage einer verstärkten Progression von AIDS

Die Vermehrung des HIV-Virus in T-Lymphozyten wird durch eine Aktivierung von Chemokinrezeptoren, deren Wirkung über die Aktivierung von G-Proteinen vermittelt wird, gesteigert (Kinter, A., Catanzaro, A., Monaco, J., Ruiz, M., Justement, J., Moir, S., Arthos, J., Oliva, A., Ehler, L., Mizell, S., Jackson, R., Ostrowski, M., Hoxie, J., Offord, R., and Fauci, A.S. CC-chemokines enhance the replication of T-tropic strains of HIV-1 in CD4(+) T cells: role of signal transduction.
*Proc.Natl.Acad.Sci.U.S.A.* 95(20):11880-11885, 1998). Damit ist zu erwarten, daß in T-Lymphozyten von 825T-Allelträgern, die eine gesteigerte Aktivierbarkeit von G-Proteinen aufweisen, nach HIV-Infektion eine verstärkte Virusvermehrung stattfindet. Somit haben diese Patienten ein erhöhtes Risiko, nach HIV-Infektion früher an AIDS zu erkranken als HIV-positive Patienten, die homozygot für das C825-Allel am GNB3-Locus sind. Nachfolgend ist die Genotypverteilung von 515 HIV-positiven Patienten und von 622 HIV-negativen Blutspendern dargestellt.

| | HIV positiv | HIV negativ |
|---|---|---|
| TT | 64 | 56 |
| TC | 235 | 276 |
| CC | 216 | 290 |
| Summe | 515 | 622 |
| T-Allelfrequenz | 35,2 % | 31,2 % |

Es findet sich ein signifikanter Unterschied der Genotypverteilung zwischen gesunden Kontrollprobanden und HIV-positiven Personen (chi quadrat = 4.253, 1 Freiheitsgrad, p = 0.0392; chi-square test for trend). Das Risiko für den TT- *versus* den CC-Genotyp, HIV-positiv zu sein beträgt damit 1,5 (1,0 - 2,3; p= 0.035; chi Quadrat = 4.4).

Es findet sich eine weitere Akkumulation des 825T-Allels innerhalb der Gruppe von HIV-positiven Patienten die an AIDS erkrankt sind, bzw. deren Anzahl CD4-positiver Zellen unter 200 pro µl Blut abgesunken ist.

| | HIV positiv, CD4 < 200 Zellen/ µl Blut | HIV positiv, CD4 ≥ 200 Zellen / µl Blut |
|---|---|---|
| TT | 40 | 14 |
| TC | 122 | 89 |
| CC | 113 | 80 |
| Summe | 275 | 183 |
| T-Allelfrequenz | 36,7 % | 32 % |

Damit besteht bei HIV-positiven Patienten, die ein 825T-Allel tragen, ein erhöhtes Risiko, an AIDS zu erkranken. Für homozygote 825T-Allelträger ist das Risiko gegenüber homozygoten C825-Allelträgern 2-fach erhöht (OR TT/CC = 2,0 (1,0 - 3,9; p < 0,05)).

Eine Genotypisierung am GNB3-Locus bietet also die Möglichkeit, daß HIV-positiven 825T-Allelträgern ein erhöhtes Risiko zugeordnet wird, eine verstärkte Progression der Erkrankung zu zeigen, wobei es insbesondere zu einer verstärkten Vermehrung des AIDS-Virus kommt. Ferner ist das Risiko eines schnelleren Absinkens der CD4-Zellen damit verbunden.

Im Rahmen der Infektion mit dem HIV-Virus (sexueller Übertragungsweg) kommt es zumeist zunächst zum Befall von Makrophagen, Monozyten und Langerhanszellen. Die sogenannten "M-tropen" "R5" HIV-Viren benutzen zum Eintritt in diese Zellen u.a. einen Chemokinrezeptor vom Typ CCR5. Individuen, bei denen eine homozygote CCR5Δ32-Deletion nachzuweisen ist, haben ein vermindertes Risiko für eine HIV-Infektion. Personen, bei denen das CCR5Δ32-Allel in heterozygoter Form vorliegt, zeigen einen verlängerten Zeitraum von der HIV-Infektion bis zur Serokonversion, bzw. eine verzögerte Progression der Erkrankung. (1. Quillent, C., Oberlin, E., Braun, J., Rousset, D., Gonzalez-Canali, G., Metais, P., Montagnier, L., Virelizier, J.L., Arenzana-Seisdedos, F., and Beretta, A. HIV-1-resistance phenotype conferred by combination of two separate inherited mutations of CCR5 gene. *Lancet* 351(9095):14-18, 1998; 2. Mummidi, S., Ahuja, S.S., Gonzalez, E., Anderson, S.A., Santiago, E.N., Stephan, K.T., Craig, F.E., O'Connell, P., Tryon, V., Clark, R.A., Dolan, M.J., and Ahuja, S.K. Genealogy of the CCR5 locus and chemokine system gene variants associated with altered rates of HIV-1 disease progression. *Nat.Med.* 4(7):786-793, 1998; 3. Magierowska, M., Theodorou, I., Debre, P., Sanson, F., Autran, B., Riviere, Y., Charron, D., and Costagliola, D. Combined genotypes of CCRS, CCR2, SDF1, and HLA genes can predict the long-term nonprogressor status in human immunodeficiency virus-1- infected individuals. *Blood* 93(3):936-941, 1999; 4. Michael, N.L., Louie, L.G., Rohrbaugh, A.L., Schultz, K.A., Dayhoff, D.E., Wang, C.E., and Sheppard, H.W. The role of CCR5 and CCR2 polymorphisms in HIV-1 transmission and disease progression [see comments]. *Nat.Med*. 3(10):1160-1162, 1997; 5. Fauci, A.S. Host factors and the pathogenesis of HIV-induced disease. Nature 384:529-534, 1996.)

Im Gegensatz dazu verstärkt die Gegenwart einer Variante im CCRS-Promoter (CCR5P1) die AIDS-Progression, insbesondere bei homozygoten CCR5P1-Trägern. (Martin, M.P., Dean, M., Smith, M.W., Winkler, C., Gerrard, B., Michael, N.L., Lee, B., Doms, R.W., Margolick, J., Buchbinder, S., Goedert, J.J., O'Brien, T.R., Hilgartner, M.W., Vlahov, D., O'Brien, S.J., and Carrington, M. Genetic acceleration of AIDS progression by a promoter variant of CCR5. *Science* 282(5395):1907-1911, 1998.)

Im weiteren Verlauf der Erkrankung kommt es zu Veränderung der Virusart in der Weise, daß die sogenannten "T-tropen" (X4)-Viren überwiegen, welche dann vorwiegend CD4-positive T-Lymphozyten befallen. Der Eintritt dieser Viren erfolgt über den G-Proteingekoppelten CXCR4 - Chemokinrezeptor, zu dessen natürlichen Liganden u.a. Stromal Cell Derived Factor 1 alpha (SDF-1α) gehört. Eine Reihe von Chemokinen (SDF-1α, RANTES, etc.) stimulieren die Vermehrung von T-tropen Viren in CD4-positiven T-Zellen, wobei der Signalübertragung über Pertussistoxin-sensitiven G-Proteinen eine entscheidende Bedeutung zukommt: Eine Hemmung der G-Proteinaktivierung durch Inkubation von Zellen mit Pertussistoxin reduziert die Virusvermehrung insbesondere bei niedriger Viruszahl. (Kinter, A., Catanzaro, A., Monaco, J., Ruiz, M., Justement, J., Moir, S., Arthos, J., Oliva, A., Ehler, L., Mizell, S., Jackson, R., Ostrowski, M., Hoxie, J., Offord, R., and Fauci, A.S. CC-chemokines enhance the replication of T-tropic strains of HIV-1 in CD4(+) T cells: role of signal transduction. *Proc.Natl.Acad.Sci.U.S.A*. 95(20):11880-11885, 1998.)

Umgekehrt läßt sich die Schlußfolgerung ziehen, daß bei Vorliegen eines 825T-Allels, welches die Expression von G β3-s und Gβ3-s2 und eine verstärkte Aktivierbarkeit von G-Proteinen anzeigt, die Vermehrung solcher Viren und damit die AIDS - Progression gesteigert sein sollten.

Eine Veränderung im Gen, welches für SDF-1 kodiert (G→A - Transition bei Position 801, gezählt vom Startkodon) wird als SDF1-3'UTR-801G-A oder als SDF1-3'A bezeichnet. Homozygote SDF1-3'A zeigen eine verminderte Progression zu AIDS. (Winkler, C., Modi, W., Smith, M.W., Nelson, G.W., Wu, X., Carrington, M., Dean, M., Honjo, T., Tashiro, K., Yabe, D., Buchbinder, S., Vittinghoff, E., Goedert, J.J., O'Brien, T.R., Jacobson, L.P., Detels, R., Donfield, S., Willoughby, A., Gomperts, E., Vlahov, D., Phair, J., and O'Brien, S.J. Genetic restriction of AIDS pathogenesis by an SDF-1 chemokine gene variant. ALIVE Study, Hemophilia Growth and Development Study (HGDS), Multicenter AIDS Cohort Study (MACS), Multicenter Hemophilia Cohort Study (MHCS), San Francisco City Cohort (SFCC) [see comments]. *Science* 279 (5349) :389-393, 1998.)

Zum Nachweis einer verstärkten AIDS-Progression von 825T-Allelträgern wurden 690 HIV-positive, homo- und heterosexuelle Personen (Männer und Frauen) untersucht, bei den das HIV-Virus auf sexuellem Wege übertragen wurde. Nach Genotypisierung wurden die folgenden Endpunkte der Erkrankung festgelegt, die eine mögliche Definition der Erkrankung an AIDS darstellen:
1. AIDS. Hierbei ist AIDS definiert als AIDSdefinierende Erkrankung oder CD4 -Zellzahl < 200. Diese AIDS - Definition entspricht der 1993 vom Center for Disease Control (CDC; Atlanta, USA) revidierten AIDS- Definition.
2. CD4 - Zellzahl < 200
3. Minimale CD4-Zellzahl
4. Maximale Viruslast

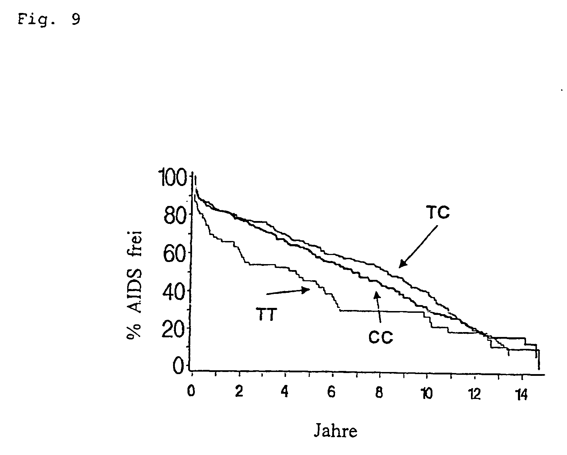

In Fig. 9 ist der Zeitpunkt vom ersten positiven HIV-Test bis zur AIDS- Diagnose als Kaplan- Meier- Kurven in Abhängigkeit vom Genotyp dargestellt. Fig. 9 zeigt, daß homozygote 825T-Allelträger den Endpunkt AIDS gemäß der CDC - Definition von 1993 signifikant früher erreichen, als homozygote oder heterozygote 825C-Allelträger.

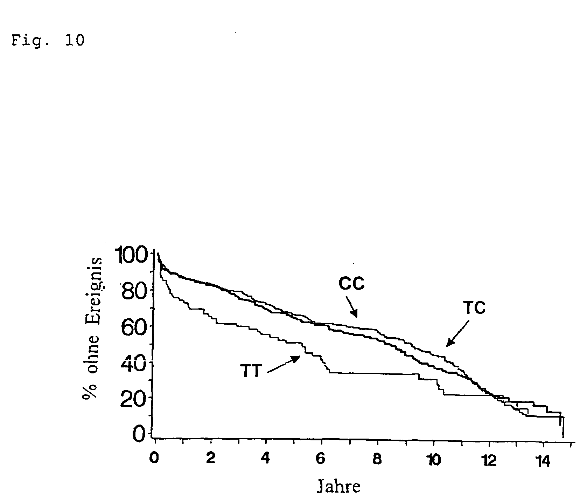

Fig. 10 zeigt den Zeitraum zwischen erstem positiven HIV - Test und Absinken der CD4-Zellzahl unter 200. Hier ist der Zeitverlauf für homozygote 825T-Allelträger gegenüber homo- und heterozygoten C825-Allelträgern ebenfalls drastisch beschleunigt.

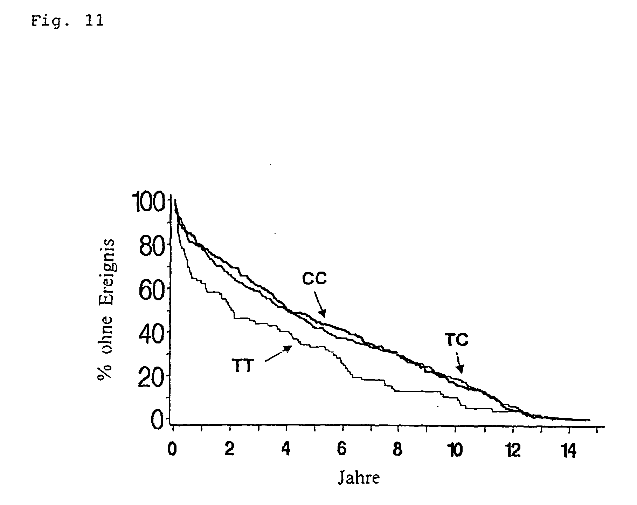

Fig. 11 zeigt den Zeitverlauf von erstem positivem HIV - Test und individuell niedrigster CD4-Zellzahl. Hier ergibt sich ebenfalls eine signifikante Beschleunigung bei homozygoten 825T-Allelträgern gegenüber homo- und heterozygoten C825-Allelträgern.

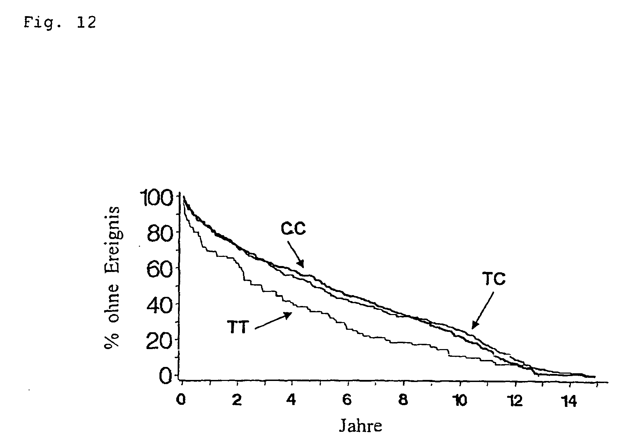

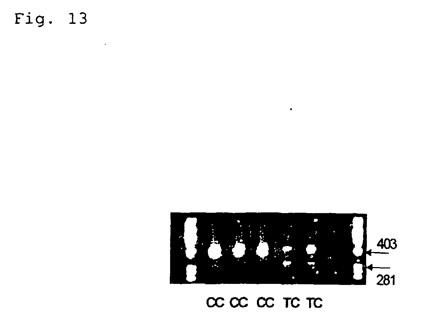

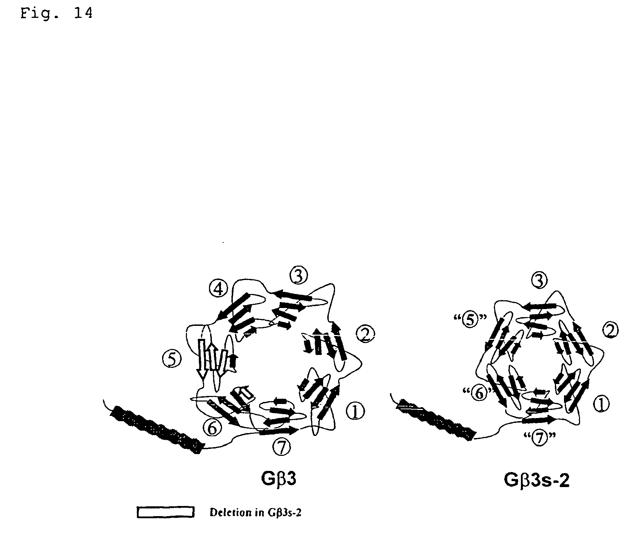

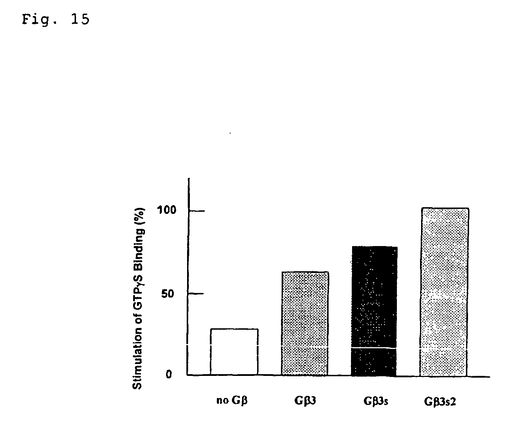

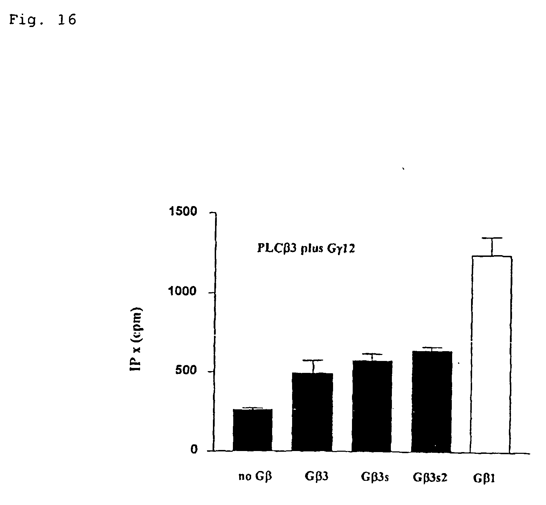

In Fig. 12 ist der Zeitraum zwischen erstem positiven HIV-Test und maximaler Viruslast dargestellt. Wiederum zeigt sich, daß 825T-Allelträger ein deutlich erhöhtes Risiko aufweisen, früher eine maximale Viruslast aufzuweisen als homo- oder heterozygote C825-Allelträger.

### 7.1 Korrelation zum CCR5-Allelstatus

Nachfolgend wurde zusätzlich der CCR5-Allelstatus bezüglich des Vorhandenseins der Δ32-Deletion untersucht. Die in der nachfolgenden Tabelle dargestellten relativen Risiken (RH) werden zusammen mit den 95 % Konfidenzintervallen (95 % CI) dargestellt.

Ohne Beachtung des genetischen Hintergrunds ist das Risiko für AIDS oder Absinken der CD4-Zellzahl < 200 für homozygote 825T-Allelträger gegenüber homo- und heterozygoten C825-Allelträgern etwa verdoppelt. Ohne Berücksichtigung des genetischen Hintergrundes findet man ein 1,4-fach erhöhtes Risiko für CCR5Δ32 -Allelträger im Beobachtungszeitraum eine CD4-Zellzahl < 200 zu erreichen. Nachfolgend wird der Einfluß des 825T-Allels für CCR5-Wildtyp und für CCR5Δ32 getrennt untersucht. Bei Vorhandensein des homozygotem CCR5-Wildtyp beträgt das Risiko für AIDS oder CD4-Zellzahl < 200 für homozygote 825T-Allelträger gegenüber heterozygoten C825-Allelträgern etwa 1,6.

Bei Vorhandensein des CCR5Δ32 Genotyps, welcher ursprünglich als protektiv beschrieben wurde, findet man jedoch für homozygote 825T-Allelträger eine weitere Erhöhung des Risikos auf nahezu das 3-fache gegenüber heterozygoten C825-Allelträgern.

**Tabelle : GNB3 825 Genotypstatus und CCR5 -Genotypstatus und AIDS-Progression COX Proportional Hazard Modell:**

| Konstant | Zielgröße | Genstatus untersucht | RH | 95% CI |
|---|---|---|---|---|
| nichts | AIDS | GNB3 TT versus TC+CC | 1.9 | (1.4-2.6) |
| | CD4 < 200 | " | 1.9 | (1.4-2.6) |
| | CD4 min | " | 1.5 | (1.2-2.0) |
| | PCR max | " | 1.5 | (1.2-2.0) |
| | | | | |
| nichts | AIDS | CCR5 WT versus CCR5Δ 32 | ns | |
| | CD4 < 200 | " | 1.4 | (1.0-1.9) |
| | CD4 min | " | ns | |
| | PCR max | " | ns | |
| | | | | |
| CCR 5 = WT | AIDS | GNB3 TT versus TC+CC | 1.6 | (1.1-2.3) |
| | CD4 < 200 | " | 1.6 | (1.1-2.3) |
| | CD4 min | " | 1.4 | (1.1-1.9) |
| | PCR max | " | 1.4 | (1.0-1.9) |
| | | | | |
| CCR5 Δ32 | AIDS | GNB3 TT versus TC + CC | 2.7 | (1.3 - 5.5) |
| | CD4 < 200 | " | 3.0 | (1.5-6.3) |
| | CD4 min | " | 2.1 | (1.1-3.8) |
| | PCR max | " | 1.9 | (1.0-3.4) |

| | | | | |
|---|---|---|---|---|
| RH = relative hazard; 95% CI = 95 % Kofidenzintervall; GNB3 = G -Protein β3-Untereinheit; TT = Homozygot für 825T; TC und CC, hetero- oder homozygot für C825; CCR5 WT = CCR5-Wildtyp (Fehlen der Δ32-Deletion); PCR-max, Zeitraum bis zur maximalen Viruslast; AIDS, Zeitraum bis zum Eintritt von AIDS gemäß CDC-Definitio von 1993; ns = nicht signifikant | | | | |

Es läßt sich daher zusammenfassend für HIV-positive Patienten feststellen:
1. Homozygotie für das 825T-Allel in GNB3 erhöht das Risiko für die Progression zu AIDS.
2. Dieser Effekt wird bei Vorliegen des CCR5Δ32-Genotyps weiter verstärkt.

### 8. Vorhersage von Osteoporose

Eine generalisierte Osteoporose stellt eine der häufigsten Erkrankungen von Frauen nach der Menopause dar und beinhaltet ein erhöhtes Risiko für Knochenfrakturen. G-Proteine sind an Prozessen, die zum Umbau des Knochens führen, maßgeblich beteiligt. Eine veränderte Aktivierbarkeit von G- Proteinen ist damit erheblich am Erkrankungsrisiko für Osteoporose beteiligt (May, L.G. and Gay, C.V. Multiple G-protein involvement in parathyroid hormone regulation of acid production by osteoclasts. J.Cell Biochem. 64(1):161-170, 1997; Gordeladze, J.O., Lund, H.W., Jablonski, G., and Bruland, O.S. Diverse expression of G-proteins in human sarcoma cell lines with different osteogenic potential: Evidence for the involvement of Gᵢ₂in cell proliferation. J.Cell.Biochem. 60:95-106, 1996).

Auch in diesem Fall zeigen Trägerinnen des GNB3-825T-Allels ein erhöhtes Risiko, an Osteoporose zu erkranken.

### 9. Vorhersage von Morbus Alzheimer

Eine geänderte Aktivierbarkeit von G-Proteinen und eine veränderte Regulation von K⁺-Kanälen wurde bei Patienten mit Morbus Alzheimer beschrieben. Ferner wurde eine verminderte Aktivierung der Adenylylcyclase nach Stimulation β- adrenerger Rezeptoren beschrieben. Diese Phänomene können auf eine erhöhte Aktivierbarkeit Pertussistoxin-sensitiver G-Proteine mit Expression der Gβ3s-Spleißvariante zurückgeführt werden (Okamoto, T., Takeda, S., Murayama, Y., Ogata, E., and Nishimoto, I. Ligand-dependent G protein coupling function of amyloid transmembrane precursor. J.Biol.Chem. 270:4205-4208, 1995; Nishimoto, I., Okamoto, T., Matsuura, Y., Takahashi, S., Murayama, Y., and Ogata, E. Alzheimer amyloid protein precursor complexes with brain GTPbinding protein G₀. Nature 362:75-79, 1993; Etcheberrigaray, R., Ito, E., Oka, K., Tofel-Grehl, B., Gibson, G.E., and Alkon, D.L. Potassium channel dysfunction in fibroblasts identifies patients with Alzheimer disease. Proc.Natl.Acad.Sci.USA 90:8209-8213, 1993; Yamatsuji, T., Matsui, T., Okamoto, T., Komatsuzaki, K., Zakeda, S., Fukumoto, H., Iwatsubo, T., Suzuki, N., Asami-Odaka, A., Ireland, S., Kinane, T.B., Giambarella, U., and Nishimoto, I. G protein-mediated neuronal DNA fragmentation induced by familial Alzheimer's disease-associated mutants of APP. Science 272:1349-1352, 1996; Cowburn, R.F., Wiehager, B., Ravid, R., and Winblad, B. Acetylcholine muscarinic M2 receptor stimulated [³⁵S]GTPγS binding shows regional selective changes in Alzheimer's disease postmortem brain. Neurodegeneration 5:19-26, 1996).

Demzufolge weisen Träger des GNB3-825T-Allels ein erhöhtes Risiko auf, an Morbus Alzheimer zu erkranken. Zudem trägt die bei Trägern des GNB3-825T-Allels zu beobachtende früh einsetzende unter 3. beschriebene Atherosklerose zur Entstehung des Morbus Alzheimer bei.

### 10. Vorhersage einer erektilen Dysfunktion (Impotenz)

Die Erektion des Penis nach sexueller Stimulation wird durch einen gesteigerten Blutzufluß bei gleichzeitig vermindertem Blutabfluß hervorgerufen. Die für den gesteigerten Blutzufluß erforderlichen Mechanismen umfassen die Wirkungen von Hormonen, deren Wirkung über G-Proteine vermittelt wird.

Es wurden 63 Männer mit nachgewiesener erektiler Dysfunktion und 614 gesunde, männliche Kontrollen am GNB3 825-Locus genotypisiert:

| | Erektile Dysfunktion | Kontrollmänner |
|---|---|---|
| TT | 2 (3) | 55 (9) |
| TC | 20 (32) | 275 (45) |
| CC | 41 (65) | 284 (46) |
| Summe | 63 | 614 |
| FT (%) | 19,0 % | 31,4 % |

Die Zahlen sind n (%)

Die Verteilung der Genotypen ist signifikant verschieden (chi-Quadrat = 8,7; 2 Freiheitsgrade, p = 0,01), wobei bei den Männern mit erektiler Dysfunktion eine drastische Reduktion der Frequenz des 825T-Allels auf 19 % auffällt. Hierdurch lassen sich für die erektile Dysfunktion im Vergleich zu einer randomisierten Kontrollgruppe die folgenden Risiken (odds ratios; OR) errechnen:
CC/TT, OR = 4,0 (95 % Cl: 0,9 - 16,9; p = 0,04)
CC/TC, OR = 2,0 (95 % Cl: 1,1 - 3,5; p = 0,01).

Damit haben homozygote C825-Allelträger gegenüber homozygoten 825T-Allelträgern ein 4-fach erhöhtes Risiko, gegenüber heterozygoten 825T-Alleträgern ein verdoppeltes Risiko, an einer erektilen Dysfunktion (Impotenz) zu erkranken. Ferner ist das Risiko für heterozygote 825T-Allelträger gegenüber homozygoten 825T-Allelträgern etwa verdoppelt.

### 11. Vorhersage von Schilddrüsenfunktionsstörungen

Träger des 825T-Allels weisen häufig eine gestörte Schilddrüsenfunktion auf und müssen mit Schilddrüsenhormonen (z. B. Thyroxin) behandelt werden.

### 12. Vorhersage eines erhöhten Schwangerschaftsrisikos

Hochdruck, Ödembildung und das sog. "HELLP-Syndrom" bedeuten eine schwerwiegende Gefahr für die Schwangerschaft, sowohl für das Leben der Mutter als auch für das Leben des ungeborenen Kindes. Es wurde gefunden, daß bei Trägerinnen des 825T-Allels, die einen Schwangerschaftshochdruck entwickeln (Gestose, Präeklampsie) ein hohes Risiko für eine Frühgeburt (Geburt vor der 37. Schwangerschaftswoche oder Geburtsgewicht des Kindes kleiner als 2500g, siehe auch 14.) besteht, wobei für diese Kinder zusätzlich das Risiko für eine Totgeburt oder für einen Tod nach der Entbindung besteht. Gleichzeitig erhöht sich bei Frauen, die Trägerinnen des 825T-Allels sind und unter Schwangerschaftsgestose leiden, das Risiko für spontane Aborte (habituelle Aborte).

Der Nachweis eines 825T-Allels ist daher geeignet, ein erhöhtes Todesrisiko für das ungeborene Kind von Schwangeren mit Schwangerschaftsgestose vorherzusagen.

Die Schwangerschaftsgestose (Präeklampsie) ist eine schwerwiegende Erkrankung, die mit Bluthochdruck, Ödemen und Proteinausscheidung einhergeht. Die Gestose ist mit einem erheblichen Risiko für die Schwangere, insbesondere aber für das ungeborene Kind verbunden. Es wurden 188 Frauen ohne Schwangerschaftsgestose und 191 Frauen mit Schwangerschaftsgestose untersucht. Dabei wurde der Genstatus am GNB3-Locus und der Genstatus bezüglich der Glu298Asp - Variante im Gen untersucht, das für die endotheliale NO-Synthase (eNOS) kodiert. (Yoshimura et al. "A missense Glu298Asp variant in the endothelial nitric oxide synthase gene is associated with coronary spasm in the Japanese", Hum Genet. 1998 Jul;103(1):65-9.)

Bei gleichzeitigem Vorliegen eines 825T-Allels in GNB3 (TC-oder TT-Genotyp) führt das homozygote Vorliegen der 298Asp-Variante in eNOS zu einem 10-fach gesteigerten Risiko für eine Schwangerschaftsgestose. Bei gleichzeitigem Vorliegen eines 825T-Allels in GNB3 (TC-oder TT-Genotyp) führt das heterozygote Vorliegen der 298Asp-Variante in eNOS zu einem 2-fach gesteigerten Risiko für eine Schwangerschaftsgestose.

### 13. Vorhersage eines niedrigen Geburtsgewichts

Bekannterweise besteht ein empirischer Zusammenhang, der eine inverse Relation zwischen Geburtsgewicht und dem Risiko beschreibt, im Laufe des Lebens an Übergewicht, Hypertonie, oder Typ-2- Diabetes zu erkranken. Hierbei wurde beschrieben, daß Individuen mit sehr niedrigem Geburtsgewicht besonders zu diesen Erkrankungen neigen. Es wurde deshalb untersucht, ob für Kinder mit 825T-Allel ein erhöhtes Risiko besteht, mit niedrigem Geburtsgewicht auf die Welt zu kommen. Als niedriges Geburtsgewicht wurde dabei dasjenige Gewicht definiert, das sich nach Aufteilung aller Gewichte im niedrigsten Quartil der gesamten Verteilung befindet. Vergleicht man die Verteilung der Genotypen am GNB3-Locus zwischen dem 1. Quartil mit den zusammengefaßten Quartilen 2 - 4 so zeigt sich für homozygote 825T-Allelträger gegenüber homozygoten C825-Allelträgern ein 6-fach erhöhtes Risiko (95% CI = 1,3 - 28,6; p < 0.05) für niedriges Geburtsgewicht (Quartil 1 versus Quartile 2-4) und für heterozygote 825T-Allelträger ein 2,4-fach erhöhtes Risiko (95 % CI = 0,7 - 7,9).

**G β3 Gen 825T - Allel und Geburtsgewicht**

| | 1. Quartil | 2. Quartil | 3. Quartil | 4. Quartil | 2.-4.Quartil |
|---|---|---|---|---|---|
| Geburtsgewicht | -3130 g | -3430 g | -3750 g | > 3750 g | Alle > 3130 g |
| TT | 5 | 1 | 2 | 2 | 5 |
| TC | 11 | 9 | 11 | 7 | 27 |
| CC | 5 | 11 | 8 | 11 | 30 |
| Summe | 21 | 21 | 21 | 20 | 62 |
| % T | 50 | 26 | 36 | 27,5 | 30 |

Nach einer Genotypisierung am GNB3-Locus wird also Trägern eines 825T-Allels ein erhöhtes Risiko zugeordnet, mit niedrigem Geburtsgewicht geboren zu werden und einer intrauterinen Wachstumsretardierung zu unterliegen.

### 14. Pharmakogenetik

Ein weiterer Gegenstand der vorliegenden Erfindung ist die Pharmakogenetik des GNB3-825T-Allels, d.h. die Möglichkeit, anhand des Genotyps die Wirkung von Pharmaka vorherzusagen.

Die meisten Pharmaka (Hormone, Rezeptoragonisten) üben ihre Wirkung über Rezeptoren aus, die an G-Proteine koppeln. Antagonisten blockieren die Hormon- Rezeptor-Interaktion.

Es hat sich nun herausgestellt, daß die Genotypisierung am GNB3-Locus dazu geeignet ist, die Wirksamkeit von Pharmaka anhand des Genotyps vorherzusagen. Dies betrifft die Ansprechbarkeit in vivo auf Hormone, Transmitter (auch Neurotransmitter) oder Pharmaka, die solche G-Protein-Heterotrimere aktivieren, welche die G-Protein-Untereinheiten Gβ3 und Gβ3s beinhalten. Damit einher geht die Vorhersage einer verminderten Wirksamkeit von Hormonen, Neurotransmittern oder Pharmaka, welche die G-Protein-Untereinheit GαS stimulieren, z.B. β-adrenerge Agonisten. Dies gilt auch bei gleichzeitiger Verwendung des Nachweises der Arg16Gly-Variante und der Gln27Glu-Variante im β2-adrenergen Rezeptor. Das Vorhandensein des GNB3-825T-Allels zeigt also eine veränderte Pharmakogenetik an und sollte bei der spezifischen Auswahl einer Therapieform (pharmakologisch oder nichtpharmakologisch) und bei der Dosierung von Pharmaka oder Hormonen bei Hypertonie, Diabetes mellitus, koronarer Herzkrankheit, akutem Myokardinfarkt mit oder ohne Herzrhythmusstörungen, Herzrhythmusstörungen, Transplantatabstossung, erektiler Dysfunktion etc. berücksichtigt werden.

### 14.1 Erythropoetin

Im diesem Zusammenhang erlaubt das Vorhandensein des GNB3-825T-Allels aber auch die Vorhersage der Wirksamkeit der Gabe von Erythropoetin auf die Blutbildung und Vorhersage der Entstehung einer Hypertonie unter dieser Therapie sowie die Vorhersage der Gefahr, unter immunsuppressiver Therapie (z.B. mit Cyclosporin) eine Hypertonie zu entwickeln.

### 14.2 Agonisten am Serotonin-Rezeptor

Ebenfalls in diesem Zusammenhang kann die Wirksamkeit von Substanzen zu Therapie und Prophylaxe des Migräneanfalls (Agonisten am Serotonin-Rezeptor) vorhergesagt werden.

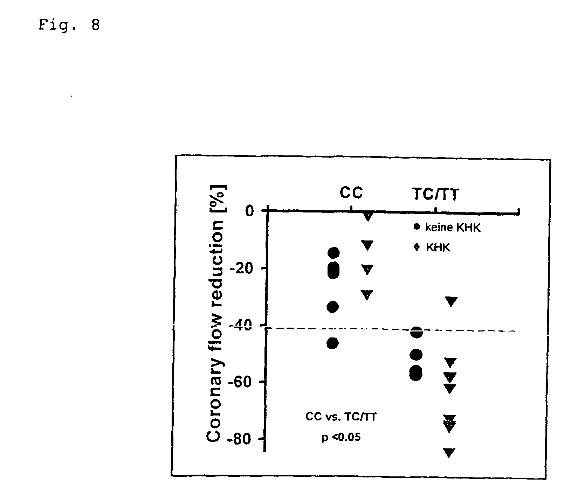

Dies wird anhand des folgenden Beispiels demonstriert. Hierbei wurde bei Probanden intrakoronar ein α2-adrenerger Agonist (BHT 933) appliziert, der zur Kontraktion von Koronargefäßen führt. Dieser Effekt wurde über die Flußänderung durch diese Gefäßabschnitte quantifiziert. Wie Fig. 8 zeigt, beobachtet man eine verstärkte Abnahme der Koronardurchblutung bei Trägern des GNB3-825T-Allels, unabhängig davon, ob sie an einer koronaren Herzkrankheit (KHK) erkrankt sind. Das heißt, daß bei Trägern des GNB3-825T-Allels eine Verstärkung der Wirkung solcher Pharmaka vorauszusagen ist.

### 14.3 β-Adrenozeptorblocker

Als weiteres Beispiel für den Einsatz einer Genotypisierung am GNB3-Locus ist die Vorhersage der Wirksamkeit von Substanzen zu benennen, welche β-adrenerge Rezeptoren blockieren. Hier ist anzuführen, daß junge, gesunde 825T-Allelträger (homo- oder heterozygot) im Vergleich zu homozygoten C825-Allelträgern ein erhöhtes Schlagvolumen des Herzens aufweisen (TC/TT = 92,9 ± 4,1 ml (n=30); CC = 74,7 ± 4,0 ml (n=19);p<0,01). Nach intravenöser Gabe des β-Adrenozeptorblockers Propanolol verringert sich das Schlagvolumen im Mittel um 3 ml bei homozygoten C825-Allelträgern, hingegen um 12 ml bei homo- und heterozygoten 828T-Allelträgern (p<0,05). In gleicher Weise findet sich eine verstärkte Verringerung des Herzzeitvolumens bei 825T-Allelträgern. Somit kann mittels Feststellung des GNB3 C825T-Status die pharmakologisch-physiologische Wirkung einer Blockade von β-adrenergen Rezeptoren vorhergesagt werden. Dies bezieht sich nicht nur auf nicht-selektive β-Blocker wie das genannte Propanolol, sondern auf alle β-Adrenozeptorblocker, also auch selektive β1- und β2-Rezeptorblocker.

### 14.4 Prostaglandin E1

Bei Vorliegen einer erektilen Dysfunktion erfolgt zur diagnostischen Abklärung, aber auch, um möglicherweise eine Dauertherapie einzuleiten, eine Injektion von Prostaglandin E1 ins corpus cavernosum. Prostaglandin E1 aktiviert die Adenylyzyclase, und die nachfolgende Bildung von cAMP relaxiert glatte Gefäßmuskelzellen und induziert damit einen erhöhten arteriellen Blutenstrom und damit die Erektion des Penis. Der Grad der erfolgenden Erektion kann über ein Punktesystem (Score 0-5) quantifiziert werden. Hierbei entsprechen die Scores 4 und 5 einer suffizienten, für eine Penetration ausreichende Erektion, während Scores < 4 als nicht ausreichend zu betrachten sind.

Bei 87 Männern mit erektiler Dysfunktion erfolgte eine Gabe von 10µg Prostaglandin E1 mit Quantifizierung des Erektions-Scores. Hierbei ergab sich folgende Verteilung der Genotypen: Ausreichende Erektion (Scores 4 und 5): TT=3; TC=15; CC=16; (Frequenz des GNB3 825T-Allels: 30.9 %). Unzureichende oder fehlende Erektion: TT=3; TC=16; CC=34; (Frequenz des GNB3 825T-Allels: 20.8 %). In ähnlicher Weise findet man bei homzygoten C825-Allelträgern nach Injektion von Prostaglandin E1 einen verminderten Anstieg der dopplersonographisch gemessenen arteriellen Durchblutung.

Somit läßt sich homozygoten 825T-Allelträgern ein ca. verdoppeltes Risiko zuordnen, auf die Injektion mit Prostaglandin E1 nicht mit einer ausreichenden Erektion zu reagieren.

### Anlage 1: Lokalisation des Polymorphismus in cDNA

### SEQ ID No 1: β3-Originalsequenz von Levine

Die Exons sind wechselweise unterstrichen. Der Bereich, der durch kryptisches Splicen wegfällt, ist fettgedruckt.

1 gggtcgATGG GGGAGATGGA GCAACTGCGT CAGGAAGCGG AGCAGCTCAA GAAGCAGATT
Start-ATG EXON 3 nach Ansari-Lari
Nucleotide 1-6 scheinen nicht zuzutreffen

61 GCAGATGCCA GGAAAGCCTG TGCTGACGTT ACTCTGGCAG AGCTGGTGTC TGGCCTAGAG
/Beginn EXON 4 /EXON 5 Beginn
==> ENDE 1 KLON ANSARI
121 GTGGTGGGAC GAGTCCAGAT GCGGACGCGG CGGACGTTAA GGGGACACCT GGCCAAGATT
EXON 5

181 TACGCCATGC ACTGGGCCAC TGATTCTAAG CTGCTGGTAA GTGCCTCGCA AGATGGGAAG
EXON 5 / Beginn EXON 6

241 CTGATCGTGT GGGACAGCTA CACCACCAAC AAGGTGCACG CCATCCCACT GCGCTCCTCC
EXON 6 / EXON 7

301 TGGGTCATGA CCTGTGCCTA TGCCCCATCA GGGAACTTTG TGGCATGTGG GGGGCTGGAC
EXON 7

361 AACATGTGTT CCATCTACAA CCTCAAATCC CGTGAGGGCA ATGTCAAGGT CAGCCGGGAG
EXON 7

421 CTTTCTGCTC ACACAGGTTA TCTCTCCTGC TGCCGCTTCC TGGATGACAA CAATATTGTG
EXON 7 /EXON 8

481 ACCAGCTCGG GGGACACCAC GTGTGCCTTG TGGGACATTG AGACTGGGCA GCAGAAGACT
EXON 8 /EXON 9

kryptisches SPLICING
541 GTATTTGTGG GACACACGGG TGACTGCATG AGCCTGGCTG TGTCTCCTGA CTTCAATCTC
EXON 9
kryptisches SPLICING

601 TTCATTTCGG GGGCCTGTGA TGCCAGTGCC AAGCTCTGGG ATGTGCGAGA GGGGACCTGC
EXON 9
kryptisches SPLICING /

661 CGTCAGACTT TCACTGGCCA CGAGTCGGAC ATCAACGCCA TCTGTTTCTT CCCCAATGGA
EXON 9 / Beginn EXON 10

721 GAGGCCATCT GCACGGGCTC GGATGACGCT TCCTGCCGCT TGTTTGACCT GCGGGCAGAC
EXON 10

781 CAGGAGCTGA TCTGCTTCTC CCACGAGAGC ATCATCTGCG GCATCACGTC CGTGGCCTTC
EXON 10 Polymorphismusstelle: acgtc tgt

841 TCCCTCAGTG GCCGCCTACT ATTCGCTGGC TACGACGACT TCAACTGCAA TGTCTGGGAC
EXON 10

901 TCCATGAAGT CTGAGCGTGT GGGCATCCTC TCTGGCCACG ATAACAGGGT GAGCTGCCTG
EXON 10 /Beginn EXON 11

961 GGAGTCACAG CTGACGGGAT GGCTGTGGCC ACAGGTTCCT GGGACAGCTT CCTCAAAATC
EXON 11

1021 TGGAACTGAg gaggctggag aaagggaagt ggaaggcagt gaacacactc agcagccccc
EXON 11
End of Open Reading Frame

1081 tgcccgaccc catctcattc aggtgttctc ttctatattc cgggtgccat tcccactaag
EXON 11

1141 ctttctcctt tgagggcagt ggggagcatg ggactgtgcc tttgggaggc agcatcaggg
EXON 11

Ban Polymorphism

1201 acacaggggc aaagaactgc cccatctcct cccatggcct tccctcccca cagtcctcac
EXON 11

1261 agcctctccc ttaatgagca aggacaacct gcccctcccc agccctttgc aggcccagca
EXON 11

1321 gacttgagtc tgaggcccca ggccctagga ttcctccccc agagccacta cctttgtcca
EXON 11

tctggcac[?]a cta
1381 ggcctgggtg gtatagggcg tttggccctg tgactatggc tctggcacca ctagggtcct
EXON 11

1441 ggccctcttc ttattcatgc tttctccttt ttctaccttt ttttctctcc taagacacct
EXON 11

1501 gcaataaagt gtagcacccct ggt
EXON 11 POLY A SITE

### Anlage 2

SEQ ID No 2: Sequenz mit den beiden Polymorphismen (Numerierung nach der Levine-Sequenz)

1 gggtcgATGG GGGAGATGGA GCAACTGCGT CAGGAAGCGG AGCAGCTCAA GAAGCAGATT
61 GCAGATGCCA GGAAAGCCTG TGCTGACGTT ACTCTGGCAG AGCTGGTGTC TGGCCTAGAG
121 GTGGTGGGAC GAGTCCAGAT GCGGACGCGG CGGACGTTAA GGGGACACCT GGCCAAGATT
181 TACGCCATGC ACTGGGCCAC TGATTCTAAG CTGCTGGTAA GTGCCTCGCA AGATGGGAAG
241 CTGATCGTGT GGGACAGCTA CACCACCAAC AAGGTGCACG CCATCCCACT GCGCTCCTCC
301 TGGGTCATGA CCTGTGCCTA TGCCCCATCA GGGAACTTTG TGGCATGTGG GGGGCTGGAC
361 AACATGTGTT CCATCTACAA CCTCAAATCC CGTGAGGGCA ATGTCAAGGT CAGCCGGGAG
421 CTTTCTGCTC ACACAGGTTA TCTCTCCTGC TGCCGCTTCC TGGATGACAA CAATATTGTG
481 ACCAGCTCGG GGGACACCAC GTGTGCCTTG TGGGACATTG AGACTGGGCA GCAGAAGACT
541 GTATTTGTGG GACACACGGG TGACTGCATG AGCCTGGCTG TGTCTCCTGA CTTCAATCTC
601 TTCATTTCGG GGGCCTGTGA TGCCAGTGCC AAGCTCTGGG ATGTGCGAGA GGGGACCTGC
661 CGTCAGACTT TCACTGGCCA CGAGTCGGAC ATCAACGCCA TCTGTTTCTT CCCCAATGGA
721 GAGGCCATCT GCACGGGCTC GGATGACGCT TCCTGCCGCT TGTTTGACCT GCGGGCAGAC
781 CAGGAGCTGA TCTGCTTCTC CCACGAGAGC ATCATCTGCG GCATCACGTC TGTGGCCTTC
841 TCCCTCAGTG GCCGCCTACT ATTCGCTGGC TACGACGACT TCAACTGCAA TGTCTGGGAC
901 TCCATGAAGT CTGAGCGTGT GGGCATCCTC TCTGGCCACG ATAACAGGGT GAGCTGCCTG
961 GGAGTCACAG CTGACGGGAT GGCTGTGGCC ACAGGTTCCT GGGACAGCTT CCTCAAAATC
1021 TGGAACTGAg gaggctggag aaagggaagt ggaaggcagt gaacacactc agcagccccc
1081 tgcccgaccc catctcattc aggtgttctc ttctatattc cgggtgccat tcccactaag
1141 ctttctcctt tgagggcagt ggggagcatg ggactgtgcc tttgggaggc agcatcaggg
1201 acacaggggc aaagaactgc cccatctcct cccatggcct tccctcccca cagtcctcac
1261 agcctctccc ttaatgagca aggacaacct gcccctcccc agccctttgc aggcccagca
1321 gacttgagtc tgaggcccca ggccctagga ttcctccccc agagccacta cctttgtcca
1381 ggcctgggtg gtatagggcg tttggccctg tgactatggc tctggcacta ctagggtcct
1441 ggccctcttc ttattcatgc tttctccttt ttctaccttt ttttctctcc taagacacct
1501 gcaataaagt gtagcacccct ggt

## Patentansprüche

1. Nukleinsäuresequenz codierend für die Gβ3-Untereinheit des humanen G-Proteins mit der in SEQ ID No 2 dargestellten Sequenz.

2. Verwendung einer Genveränderung im Gen für die Gβ3-Untereinheit des humanes G-Proteins in vitro, wobei an Position 1429 in SEQ ID No 2 eine Substitution von Cytosin durch Thymin vorliegt, zur Ermittlung des Risikos, an einer mit G-Protein-Fehlsteuerung assoziierten Krankheit zu erkranken.

3. Verwendung nach Anspruch 2, wobei an Position 825 in SEQ ID No 2 eine Substitution von Cytosin durch Thymin vorliegt.

4. Verwendung nach Anspruch 2 oder 3 zur Ermittlung des Risikos, an Diabetes mellitus Typ 2, Übergewicht und Adipositas, Hypercholesterinämie, koronare Herzkrankheit, Myokardinfarkt, plötzlichem Herztod, Osteoporose, Atherosklerose, neurodegenerativen oder cerebrovaskulären Erkrankungen, insbesondere Morbus Alzheimer, einer Krankheit, die auf einer gesteigerten Reaktionsfähigkeit des Immunsystem beruht und/oder nicht an erektiler Dysfunktion zu erkranken.

5. Verwendung einer Genveränderung im Gen für die Gβ3-Untereinheit des humanen G-Proteins in vitro, wobei an Position 1429 in SEQ ID No 2 eine Substitution von Cytosin durch Thymin vorliegt, zur Vorhersage des Risikos von Frauen, an einer kardiovaskulären Erkrankung, insbesondere Bluthochdruck oder koronare Herzkrankheit zu erkranken und zur Erstellung einer gezielten Hormontherapie um das kardiovaskuläre Risiko zu vermindern.

6. Verwendung nach Anspruch 5, wobei an Position 825 in SEQ ID No 2 eine Substitution von Cytosin durch Thymin vorliegt.

7. Verfahren zur Ermittlung des Risikos eines Probanden, an einer mit G-Protein-Fehlsteuerung assoziierten Krankheit zu erkranken, bei dem die Gensequenz für die Gβ3-Untereinheit des humanen G-Proteins des Probanden mit der Gensequenz in SEQ ID No 2 in vitro verglichen wird und für den Fall, daß sie mit der Gensequenz in SEQ ID No 2 in Position 1429 übereinstimmt, dem Probanden ein erhöhtes Erkrankungsrisiko zugeordnet wird.

8. Verfahren nach Anspruch 7, bei dem für den Fall, daß die Gensequenz des Probanden mit der Gensequenz in SEQ ID No 2 in Position 825 übereinstimmt, dem Probanden ein erhöhtes Erkrankungsrisiko zugeordnet wird.

9. Verfahren nach Anspruch 7 oder 8, **dadurch gekennzeichnet, daß** zur Ermittlung des Risikos, an Diabetes mellitus Typ 2 zu erkranken, gleichzeitig Genveränderungen im IRS1-Gen (3931A-Variante; Gly971Arg), im IRS2-Gen, im Gen, das für die p85α-regulatorische Untereinheit der PI3-Kinase kodiert (1020 G →A; Kodon 326 Met →Ile), im Gen, das für den β3-adrenergen Rezeptor kodiert (Trp64Arg), im Gen, das für den β2-adrenergen Rezeptor kodiert (hier insbesondere die Arg16Gly-Variante und die Gln27Glu-Variante), im Gen, das für Tumornekrosefaktor α kodiert und/oder im Gen, das für Leptin oder den Leptinrezeptor kodiert, untersucht werden.

10. Verfahren nach Anspruch 7 oder 8, **dadurch gekennzeichnet, daß** zur Ermittlung des Risikos, an Übergewicht und Adipositas zu erkranken, gleichzeitig Genveränderungen im IRS1-Gen (3931A-Variante; Gly971Arg), im Gen, das für den β3-adrenergen Rezeptor kodiert (Trp64Arg-Variante) und/oder im Gen, das für den β2-adrenergen Rezeptor kodiert, (insbesondere die Argl6Gly-Variante und die Gln27Glu-Variante) untersucht werden.

11. Verfahren nach Anspruch 7 oder 8, **dadurch gekennzeichnet, daß** zur Ermittlung des Risikos, an koronarer Herzkrankheit und/oder Myokardinfarkt zu erkranken, gleichzeitig Genveränderungen im IRS1-Gen (3931A-Variante; Gly971Arg) untersucht werden.

12. Verfahren nach Anspruch 7 oder 8, **dadurch gekennzeichnet, daß** zur Ermittlung des Risikos, an Krankheiten, die mit einer gesteigerten Reaktionsfähigkeit des Immunsystems assoziiert sind, zu erkranken, gleichzeitig Genveränderungen im β2-adrenergen Rezeptor (insbesondere die Arg16Gly- Variante und die Gln27Glu-Variante) untersucht werden.

13. Verfahren nach Anspruch 7 oder 8, **dadurch gekennzeichnet, daß** zur Ermittlung des Risikos an Schwangerschaftsgestose zu erkranken, gleichzeitig Genveränderungen im für die endotheliale NO-Synthase kodierenden Gen (insbesondere die Glu298Asp-Variante) untersucht werden.

14. Verfahren nach Anspruch 7 oder 8, **dadurch gekennzeichnet, daß** homozygoten HIV-positiven Probanden ein erhöhtes Risiko zugeordnet wird, an AIDS zu erkranken.

15. Verfahren nach Anspruch 14, **dadurch gekennzeichnet, daß** zur Ermittlung des Risikos, an AIDS zu erkranken, gleichzeitig Genveränderungen im CCR5-Gen untersucht werden und daß den homo oder heterozygoten Probanden für den CCR5Δ32-Polymorphismus ein weiter erhöhtes Risiko zugeordnet wird, an AIDS zu erkranken.

16. Verfahren nach Anspruch 14 oder 15, **dadurch gekennzeichnet, daß** zur Ermittlung des Risikos, an AIDS zu erkranken, gleichzeitig Genveränderungen im CCR5-Gen untersucht werden und daß den Probanden die das CCR5P1-Allel tragen ein weiter erhöhtes Risiko zugeordnet wird, an AIDS zu erkranken.

17. Verfahren nach Anspruch 14, **dadurch gekennzeichnet, daß** zur Ermittlung des Risikos an AIDS zu erkranken, gleichzeitig bezüglich des SDF1-3'UTR-801G-A Polymorphismus untersucht werden und daß den Probanden, die das SDFl-3'A-Allel tragen ein weiter erhöhtes Risiko zugeordnet wird, an AIDS zu erkranken.

18. Verwendung einer Genveränderung im Gen für die Gβ3-Untereinheit des humanen G-Proteins in vitro, wobei an Position 1429 in SEQ ID No 2 eine Substitution von Cytosin durch Thymin vorliegt, zur Vorhersage der Ansprechbarkeit eines Probanden in vivo auf Pharmaka.

19. Verwendung nach Anspruch 18, wobei an Position 825 in SEQ ID No 2 eine Substitution von Cytosin durch Thymin vorliegt.

20. Verfahren nach Anspruch 18 oder 19, zur Vorhersage der Ansprechbarkeit eines Probanden in vivo auf Hormone, Transmitter (auch Neurotransmitter) oder Pharmaka, die solche G-Protein-Heterotrimere aktivieren, welche die G-Protein-Untereinheiten Gβ3 und Gβ3s beinhalten und/oder die G-Protein-Untereinheit GαS stimulieren.

21. Verwendung nach einem der Ansprüche 18 bis 20 zur Vorhersage der Ansprechbarkeit eines Probanden, wobei gleichzeitig die Arg16Gly-Variante und die Gln27Glu-Variante im β2-adrenergen Rezeptor nachgewiesen wird.

22. Verwendung nach einem der Ansprüche 18 bis 21zur spezifischen Auswahl einer pharmakologischen oder nichtpharmakologisch Therapieform bzw. der Dosierung von Pharmaka oder Hormonen bei Hypertonie, Diabetes mellitus, koronarer Herzkrankheit, akutem Myokardinfarkt mit oder ohne Herzrhythmusstörungen, Herzrhythmusstörungen und Transplantatabstossung.

23. Verwendung nach einem der Ansprüche 18 bis 21, zur Vorhersage der Wirksamkeit der Gabe von Erythropoetin auf die Blutbildung und/oder Vorhersage der Entstehung einer Hypertonie unter dieser Therapie.

24. Verwendung nach einem der Ansprüche 18 bis 21, zur Vorhersage der Gefahr, unter immunsuppressiver Therapie, insbesondere mit Cyclosporin, eine Hypertonie zu entwickeln.

25. Verwendung nach einem der Ansprüche 18 bis 21 zur Vorhersage der Wirksamkeit von Substanzen zu Therapie und Prophylaxe des Migräneanfalls.

26. Verwendung einer Genveränderung im Gen für die Gβ3-Untereinheit des humanen G-Proteins in vitro, wobei an Position 1429 in SEQ ID No 2 eine Substitution von Cytosin durch Thymin vorliegt, zur Vorhersage der Ansprechbarkeit eines Probanden in vivo auf β-Adrenozeptorblocker.

27. Verwendung einer Genveränderung im Gen für die Gβ3-Untereinheit des humanen G-Proteins in vitro, wobei an Position 1429 in SEQ ID No 2 eine Substitution von Cytosin durch Thymin vorliegt, zur Vorhersage der Ansprechbarkeit eines Probanden in vivo auf Substanzen mit einer Prostaglandin E1-Wirkung, insbesondere Prostaglandin E1.

28. Verwendung nach Anspruch 26 oder 27, wobei an Position 825 in SEQ ID No 2 eine Substitution von Cytosin durch Thymin vorliegt.

29. Verwendung einer Nukleinsäuresequenz, die komplementär zu der Nukleinsäuresequenz nach Anspruch 1 oder 2 ist, zur Herstellung eines Antisense-Arzneimittels zur Therapie oder Prävention von Krankheiten.

## Claims

1. Nucleic acid sequence coding for the Gbeta3 subunit of the human G protein with the sequence described in SEQ ID No 2.

2. Use of a gene change in the gene for the Gbeta3 subunit of the human G protein in vitro, whereas at position 1429 in SEQ ID No 2 there being substitution of cytosine by thymine, for determination of the risk of developing a disease associated with G protein dysregulation.

3. Use as claimed in claim 2, whereas at position 825 in SEQ ID No 2 there being substitution of cytosine by thymine.

4. Use as claimed in claim 2 or 3 for determining the risk of developing diabetes mellitus type 2, overweight and adiposity, hypercholesterolemia, coronary heart disease, myocardial infarction, sudden cardiac death, osteoporosis, atherosclerosis, neurodegenerative or cerebrovascular conditions, especially Morbus Alzheimer's disease which is based on the increased reactivity of the immune system and/or not developing an erectile dysfunction.

5. Use of a gene change in the gene for the Gbeta3 subunit of the human G protein in vitro, whereas at position 1429 in SEQ ID No 2 there being substitution of cytosine by thymine, for predicting the risk of women for developing a cardiovascular condition, especially high blood pressure or coronary heart disease or for preparing a specific hormone therapy in order to reduce the cardiovascular risk.

6. Use as claimed in claim 5, whereas at position 825 in SEQ ID No 2 there being substitution of cytosine by thymine.

7. Process for determining the risk of a proband for developing a disease associated with G protein dysregulation in which the gene sequence for the Gbeta3 subunit of the human G protein of the proband is compared with the gene sequence in SEQ ID No 2 in vitro and in case corresponding to the gene sequence in SEQ ID No 2 in position 1429, an increased risk of disease is assigned to the proband.

8. Process as claimed in claim 7, in which, in case the gene sequence of the proband corresponds to the gene sequence in SEQ ID No 2 in position 825, an increased risk of disease is assigned to the proband.

9. Process as claimed in claim 7 or 8, wherein to determine the risk of developing diabetes mellitus type 2, gene changes in the IRS1 gene (3931A variant; Gly971Arg), in the IRS2 gene, in the gene which codes for the p85 alpha regulatory subunit of PI3 kinase (1020 G -> A; codon 326 Met -> Ile), in the gene which codes for the beta3 adrenergic receptor (Trp64Arg), in the gene which codes for the beta2-adrenergic receptor (here especially Arg16Gly variant and the Gln27Glu variant), in the gene which codes for the tumor necrosis factor alpha and/or in the gene which codes for leptine or the leptine receptor, are studied at the same time.

10. Process as claimed in claim 7 or 8, wherein to determine the risk of developing overweight and adiposity, gene changes in the IRSI gene (3931A variant; Gly971Arg), in the gene which codes for the beta3 adrenergic receptor (Trp64Arg variant), and/or in the gene which codes for the beta2-adrenergic receptor (here especially Arg16Gly variant and the Gln27Glu variant) are studied at the same time.

11. Process as claimed in claim 7 or 8, wherein to determine the risk of developing coronary heart disease and/or myocardial infarction, gene changes in the IRS1 gene (3931A variant; Gly971Arg) are studied at the same time.

12. Process as claimed in claim 7 or 8, wherein to determine the risk of developing diseases which are associated with increased reactivity of the immune system, gene changes in the beta2-adrenergic receptor (here especially the Arg16Gly variant and the Gln27Glu variant) are studied at the same time.

13. Process as claimed in claim 7 or 8, wherein to determine the risk of developing pregnancy gestosis, gene changes in the gene coding for endothelial NO synthase (especially the Glu298Asp variant) are studied at the same time.

14. Process as claimed in claim 7 or 8, wherein an increased risk of developing AIDS is assigned to homozygotic HIV-positive probands.

15. Process as claimed in claim 14, wherein to determine the risk of developing AIDS, gene changes in the CCR5 gene are studied at the same time and wherein a further increased risk of developing AIDS is assigned to the homozygotic or heterozygotic probands for the CCR5Δ32 polymorphism.

16. Process as claimed in claim 14 or 15, wherein to determine the risk of developing AIDS, gene changes in the CCR5 gene are studied at the same time and wherein a further increased risk of developing AIDS is assigned to the probands which carry the CCR5P1 allele.

17. Process as claimed in claim 14, wherein to determine the risk of developing AIDS, studies are done at the same time with respect to the SDF1-3'UTR-801G-A polymorphism and wherein a further increased risk of developing AIDS is assigned to the probands which carry the SDF1-3'A allele.

18. Use of a gene change in the gene for the Gbeta3 subunit of the human G protein in vitro, whereas at position 1429 in SEQ ID No 2 there being substitution of cytosine by thymine, for predicting the responsivity of a proband in vivo to pharmaceuticals.

19. Use as claimed in claim 18, whereas at position 825 in SEQ ID No 2 there being substitution of cytosine by thymine.

20. Process as claimed in claim 18 or 19, for predicting the responsivity of a proband in vivo to hormones, transmitters (also neurotransmitters) or pharmaceuticals which activate those G protein heterotrimers which contain the G protein subunits Gbeta3 and Gbeta3 s and/or which stimulate the G protein subunit GalphaS.

21. Use as claimed in claim 18 to 20 for predicting the responsivity of a proband, whereas at the same time the Arg16Gly variant and the Gln27Glu variant being detected in the beta2 adrenergic receptor.

22. Use as claimed in claim 18 to 21 for specific choice of a pharmacological or non-pharmacological form of treatment and accordingly the dosages of pharmaceuticals or hormones in hypertension, diabetes mellitus, coronary heart disease, acute myocardial infarction with or without cardiac irregularity, cardiac irregularities, and transplant rejection.

23. Use as claimed in one of claims 18 to 21, for prediction of the effectiveness of administering erythropoietin on blood cell formation and/or the prediction of the formation of hypertension during this therapy.

24. Use as claimed in one of claims 18 to 21, for prediction of the danger of developing hypertension during immunosuppressive therapy, especially with cyclosporin.

25. Use as claimed in one of claims 18 to 21 for prediction of the effectiveness of substances for treatment and prevention of a migraine attack.

26. Use of a gene change in the gene for the Gbeta3 subunit of the human G protein in vitro, whereas at position 1429 in SEQ ID No 2 there being substitution of cytosine by thymine, for predicting the responsivity of a proband in vivo to beta-adrenoceptor blockers.

27. Use of a gene change in the gene for the Gbeta3 subunit of the human G protein in vitro, whereas at position 1429 in SEQ ID No 2 there being substitution of cytosine by thymine, for predicting of the responsivity of a proband in vivo to substances with a prostaglandin E1 effect, especially prostaglandin E1.

28. Use as claimed in claim 26 or 27, whereas at position 825 in SEQ ID No 2 there being substitution of cytosine by thymine.

29. Use of a nucleic acid sequence which is complementary to the nucleic acid sequence as claimed in claim 1 or 2, for producing an antisense pharmaceutical for treatment or prevention of diseases.

## Revendications

1. Séquence d'acide nucléique codant pour la sous-unité Gβ3 de la protéine G humaine ayant la séquence représentée en SEQ ID N°2.

2. Utilisation d'une modification génique dans le gène de la sous-unité Gβ3 de la protéine G humaine *in vitro,* où en position 1429 dans la SEQ ID N°2, est présente une substitution de la cytosine par une thymine, pour déterminer le risque de développer une maladie associée à un réglage défaillant de la protéine G.

3. Utilisation selon la revendication 2, où en position 825 dans la SEQ ID N°2, est présente une substitution de la cytosine par une thymine.

4. Utilisation selon la revendication 2 ou 3, pour déterminer le risque de développer, le diabète mellitus de type 2, le surpoids et l'adipose, l'hypercholestérolémie, une maladie cardiaque coronaire, l'infarctus du myocarde, la mort cardiaque soudaine, l'ostéoporose, l'athérosclérose, des maladies neurodégénératives et cérébrovasculaires, en particulier la maladie d'Alzheimer Morbus, une maladie qui repose sur une réactivité accrue du système immunitaire et/ou un dysfonctionnement non érectile.

5. Utilisation d'une modification génique dans le gène de la sous-unité Gβ3 de la protéine G humaine *in vitro,* où en position 1429 dans la SEQ ID N°2, est présente une substitution de la cytosine par une thymine, pour prédire le risque de développer chez la femme, une maladie cardiovasculaire, en particulier l'hypertension artérielle ou une maladie cardiaque coronaire et pour la préparation d'une hormonothérapie ciblée pour diminuer le risque cardiovasculaire.

6. Utilisation selon la revendication 5, où en position 825 dans la SEQ ID N°2, est présente une substitution de la cytosine par une thymine.

7. Procédé de détermination du risque d'un patient à développer une maladie associée à un réglage défaillant de la protéine G, dans lequel la séquence génique de la sous-unité Gβ3 de la protéine G humaine du patient est comparée à la séquence génique de SEQ ID N°2 *in vitro* et dans le cas où elle coïnciderait avec la séquence génique de SEQ ID N°2 en position 1429, on attribue au patient, un risque élevé de maladie.

8. Procédé selon la revendication 7, dans lequel dans le cas où la séquence génique du patient coïncide avec la séquence génique de SEQ ID N°2 en position 825, on attribue au patient, un risque élevé de maladie.

9. Procédé selon la revendication 7 ou 8, **caractérisé en ce que** pour la détermination du risque de développer le diabète mellitus de type 2, on examine les modifications géniques simultanées dans le gène IRS1 (variant 3931A ; Gly971Arg), dans le gène IRS2, dans le gène qui code pour la sous-unité de régulation p85α de la kinase PI3 (1020 G→A ; codon 326 Met→Ile), dans le gène qui code pour le récepteur β3-adrénergique (Trp64Arg), dans le gène qui code pour le récepteur β2-adrénergique (ici en particulier, le variant Arg16Gly et le variant Gln27Glu), dans le gène qui code pour le facteur α de nécrose tumorale et/ou dans le gène qui code pour la leptine ou le récepteur de la leptine.

10. Procédé selon la revendication 7 ou 8, **caractérisé en ce que** pour la détermination du risque de développer un surpoids et l'adipose, on examine les modifications géniques simultanées dans le gène IRS1 (variant 3931A ; Gly97lArg), dans le gène qui code le récepteur β3-adrénergique (variant Trp64Arg) et/ou dans le gène qui code le récepteur β2-adrénergique (ici en particulier, le variant Arg16Gly et le variant Gln27Glu).

11. Procédé selon la revendication 7 ou 8, **caractérisé en ce que** pour la détermination du risque de développer une maladie cardiaque coronaire et/ou un infarctus du myocarde, on examine les modifications géniques simultanées dans le gène IRS1 (variant 3931A : Gly971Arg).

12. Procédé selon la revendication 7 ou 8, **caractérisé en ce que** pour la détermination du risque de développer des maladies, qui sont associées à une réactivité accrue du système immunitaire, on examine les modifications géniques simultanées dans le gène qui code le récepteur β2-adrénergique (ici en particulier, le variant Arg16Gly et le variant Gln27Glu).

13. Procédé selon la revendication 7 ou 8, **caractérisé en ce que** pour la détermination du risque de développer des troubles de la grossesse, on examine les modifications géniques simultanées dans le gène codant la NO-synthétase endothéliale (en particulier, le variant Glu298Asp).

14. Procédé selon la revendication 7 ou 8, **caractérisé en ce que** l'on attribue aux patients homozygotes positifs HIV, un risque élevé de développer le SIDA.

15. Procédé selon la revendication 14, **caractérisé en ce que** pour la détermination du risque de développer le SIDA, on examine les modifications géniques simultanées dans le gène CCR5 et **en ce que** l'on attribue aux patients homo- ou hétérozygotes pour le polymorphisme CCR5Δ32, un autre risque élevé de développer le SIDA.

16. Procédé selon la revendication 14 ou 15, **caractérisé en ce que** pour la détermination du risque de développer le SIDA, on examine les modifications géniques simultanées dans le gène CCR5 et **en ce que** l'on attribue aux patients qui portent l'allèle CCR5P1, un autre risque élevé de développer le SIDA.

17. Procédé selon la revendication 14, **caractérisé en ce que** pour la détermination du risque de développer le SIDA, on examine simultanément le polymorphisme du SDF1-3'UTE-901G-A et **en ce que** l'on attribue aux patients qui portent l'allèle SDF1-3'A, un autre risque élevé de développer le SIDA.

18. Utilisation d'une modification génique dans le gène de la sous-unité Gβ3 de la protéine G humaine *in vitro,* où en position 1429 dans SEQ ID N°2, est présente une substitution de cytosine par une thymine, pour prédire la sensibilité in vivo à un médicament de la part d'un patient.

19. Utilisation selon la revendication 18, où en position 825 dans la SEQ ID N°2, est présente une substitution de la cytosine par une thymine.

20. Utilisation selon la revendication 18 ou 19, pour prédire la sensibilité d'un patient à une hormone, à un transmetteur (également, un neurotransmetteur) ou à un médicament, *in vivo*, lesquels activent les hétérotrimères de la protéine G, qui contient la sous-unité Gβ3 ou Gβ3s de la protéine G et/ou stimulent la sous-unité GαS de la protéine G.

21. Utilisation selon l'une des revendications 18 à 20, pour prédire la sensibilité d'un patient, où l'on détecte simultanément le variant Argl6Gly et le variant Gln27Glu de récepteur β2-adrénergique.

22. Utilisation selon l'une des revendications 18 à 21, pour le choix spécifique d'une forme thérapeutique pharmacologique ou non pharmacologique ou bien du dosage du médicament ou de l'hormone lors d'hypertonie, de diabète mellitus, de maladie cardiaque coronaire, d'infarctus du myocarde sévère avec ou sans arythmies cardiaques, troubles du rythme cardiaque et rejet de transplant.

23. Utilisation selon l'une des revendications 18 à 21, pour prédire l'activité de l'administration d'érythropoïétine sur la formation du sang et/ou pour prédire la formation d'une hypertonie sous cette thérapie.

24. Utilisation selon l'une des revendications 18 à 21, pour prédire le danger, sous thérapie immunosuppressive, en particulier avec la ciclosporine, de développer une hypertonie.

25. Utilisation selon l'une des revendications 18 à 21, pour prédire l'activité de substances pour la thérapie et la prophylaxie des migraines.

26. Utilisation d'une modification génique dans le gène de la sous-unité Gβ3 de la protéine G humaine *in vitro*, où en position 1429 dans la SEQ ID N°2, est présente une substitution de la cytosine par une thymine, pour prédire la sensibilité d'un patient *in vivo,* à un agent bloquant le récepteur β-adrénergique.

27. Utilisation d'une modification génique dans le gène de la sous-unité Gβ3 de la protéine G humaine *in vitro,* où en position 1429 dans la SEQ ID N°2, est présente une substitution de la cytosine par une thymine, pour prédire la sensibilité d'un patient *in vivo*, à des substances avec action sur la prostaglandine E1, en particulier la prostaglandine E1.

28. Utilisation selon la revendication 26 ou 27, où en position 825 dans la SEQ ID N°2, est présente une substitution de la cytosine par une thymine.

29. Utilisation d'une séquence d'acide nucléique, qui est complémentaire à la séquence d'acide nucléique de la revendication 1 ou 2, pour la préparation d'un agent pharmaceutique anti-sens pour la thérapie ou la prévention de maladies.
